# EUROPEAN PATENT SPECIFICATION

(11) **EP 1 589 990 B1**
(45) Date of publication and mention of the grant of the patent: **14.11.2012**
(21) Application number: 03745667.0
(22) Date of filing: 27.03.2003
(51) Int. Cl.: A61K 38/20, A61P 37/00, A61P 43/00

(54) **IL-21 FOR USE IN TREATING CANCER**
IL-21 ZUR VERWENDUNG IN DER BEHANDLUNG VON KREBS
IL-21 POUR UNE UTILISATION DANS LE TRAITEMENT DU CANCER

(30) Priority: 27.03.2002 US 368438 P
(43) Date of publication of application: 02.11.2005
(73) Proprietor: The Government of the United States of America, represented by The Secretary Department of Health and Human services, Rockville, MD 20852 (US)
(72) Inventor: HWU, Patrick M.D, Houston, TX 77005 (US); WANG, Gang, Montgomery Village, MD 20886 (US); LEONARD, Warren, Bethesda, MD 20817 (US); SPOLSKI, Rosanne, Ellicott City, Maryland 21042 (US); OSAKI, Katsutoshi, Kawachi-gun, Tochigi 329-0498 (JP)
(74) Representative: Grünecker, Kinkeldey, Stockmair & Schwanhäusser
(86) International application number: PCT/US2003/009707
(87) International publication number: WO 2003/082212

(56) References cited:
- WO-A-03/028630
- WO-A1-99/61617
- PARRISH-NOVAK J ET AL: "Interleukin 21 and its Receptor are Involved in NK Cell Expansion and Regulation of Lymphocyte Function" NATURE, NATURE PUBLISHING GROUP, LONDON, GB, vol. 408, no. 6808, 2 November 2000 (2000-11-02), pages 57-68, XP003016377 ISSN: 0028-0836
- ROSENBERG SA: "The Development of New Immunotherapies for the Treatment of Cancer Using Interleukin-2." ANNALS OF SURGERY, vol. 208, no. 2, August 1988 (1988-08), XP002465174
- MOCELLIN S, WANG E, MARINCOLA FM: "Cytokines and immune response in the tumor microenvironment." J IMMUNOTHER, vol. 24, no. 5, 1997, pages 392-407, XP002465173
- DATABASE BIOSIS [Online] NELSON ET AL.: 'Anti-tumor effects of interleukin 21', XP002987282 Retrieved from PREV Database accession no. 200300356682 & BLOOD vol. 100, no. 11, 16 November 2002, page 158A
- SIVAKUMAR ET AL.: 'Interleukin-21 is a T-hepler cytokine that regulates humoral immunity and cell-mediated anti-tumor responses' IMMUNOLOGY vol. 112, 2004, pages 177 - 182, XP002987283

### SEQUENCE LISTING

<110> GOVERNMENT OF THE UNITED STATES OF AMERICA, REPRESENTED BY THE SECRETARY, DEPARTMENT OF HEALTH AND HUMAN SERVICES
<120> Method for Treating Cancer in Humans
<130> 230592
<140> PCT/US03/09707
   <141> 2003-03-27
<150> 60/368,438
   <151> 2002-03-27
<160> 8
<170> PatentIn version 3.5
<210> 1
   <211> 33
   <212> DNA
   <213> Homo sapiens
<400> 1
   ccaccggcgg tacttatgag atccagtcct ggc 33
<210> 2
   <211> 27
   <212> DNA
   <213> Homo sapiens
<400> 2
   gctagctcag gaactttcac ttccgtg 27
<210> 3
   <211> 33
   <212> DNA
   <213> Mus musculus
<400> 3
   ccaccggcgg gtggcatgga gaggaccctt gtc 33
<210> 4
   <211> 27
   <212> DNA
   <213> Mus musculus
<400> 4
   gctagcctag gagagatgct gatgaat 27
<210> 5
   <211> 162
   <212> PRT
   <213> Homo sapiens
<400> 5
   Met Arg Ser Ser Pro Gly Asn Met Glu Arg Ile Val Ile Cys Leu Met
   1 5 10 15
   Val Ile Phe Leu Gly Thr Leu Val His Lys Ser Ser Ser Gln Gly Gln
   20 25 30
   Asp Arg His Met Ile Arg Met Arg Gln Leu Ile Asp Ile Val Asp Gln
   35 40 45
   Leu Lys Asn Tyr Val Asn Asp Leu Val Pro Glu Phe Leu Pro Ala Pro
   50 55 60
   Glu Asp Val Glu Thr Asn Cys Glu Trp Ser Ala Phe Ser Cys Phe Gln
   65 70 75 80
   Lys Ala Gln Leu Lys Ser Ala Asn Thr Gly Asn Asn Glu Arg Ile Ile
   85 90 95
   Asn Val Ser Ile Lys Lys Leu Lys Arg Lys Pro Pro Ser Thr Asn Ala
   100 105 110
   Gly Arg Arg Gln Lys His Arg Leu Thr Cys Pro Ser Cys Asp Ser Tyr
   115 120 125
   Glu Lys Lys Pro Pro Lys Glu Phe Leu Glu Arg Phe Lys Ser Leu Leu
   130 135 140
   Gln Lys Met Ile His Gln His Leu Ser Ser Arg Thr His Gly Ser Glu
   145 150 155 160
   Asp Ser
<210> 6
   <211> 642
   <212> DNA
   <213> Homo sapiens
<400> 6
   gctgaagtga aaacgagacc aaggtctagc tctactgttg gtacttatga gatccagtcc     60
   tggcaacatg gagaggattg tcatctgtct gatggtcatc ttcttgggga cactggtcca    120
   caaatcaagc tcccaaggtc aagatcgcca catgattaga atgcgtcaac ttatagatat    180
   tgttgatcag ctgaaaaatt atgtgaatga cttggtccct gaatttctgc cagctccaga    240
   agatgtagag acaaactgtg agtggtcagc tttttcctgt tttcagaagg cccaactaaa    300
   gtcagcaaat acaggaaaca atgaaaggat aatcaatgta tcaattaaaa agctgaagag    360
   gaaaccacct tccacaaatg cagggagaag acagaaacac agactaacat gcccttcatg    420
   tgattcttat gagaaaaaac cacccaaaga attcctagaa agattcaaat cacttctcca    480
   aaagatgatt catcagcatc tgtcctctag aacacacgga agtgaagatt cctgaggatc    540
   taacttgcag ttggacacta tgttacatac tctaatatag tagtgaaagt catttctttg    600
   tattccaagt ggaggagccc tattaaatta tataaagaaa ta                        642
<210> 7
   <211> 146
   <212> PRT
   <213> Mus musculus
<400> 7
<210> 8
   <211> 3071
   <212> DNA
   <213> Mus musculus
<400> 8
   gagaaccaga ccaaggccct gtcatcagct cctggagact cagttctggt ggcatggaga      60
   ggacccttgt ctgtctggta gtcatcttct tggggacagt ggcccataaa tcaagccccc     120
   aagggccaga tcgcctcctg attagacttc gtcaccttat tgacattgtt gaacagctga     180
   aaatctatga aaatgacttg gatcctgaac ttctatcagc tccacaagat gtaaaggggc     240
   actgtgagca tgcagctttt gcctgttttc agaaggccaa actcaagcca tcaaaccctg     300
   gaaacaataa gacattcatc attgacctcg tggcccagct caggaggagg ctgcctgcca 360
   ggaggggagg aaagaaacag aagcacatag ctaaatgccc ttcctgtgat tcgtatgaga 420
   aaaggacacc caaagaattc ctagaaagac taaaatggct ccttcaaaag atgattcatc 480
   agcatctctc ctagaacaca taggacccga agattcctga ggatccgaga agattcccga 540
   ggactgagga gacgccggac actatagacg ctcacgaatg caggagtaca tcttgcctct 600
   tgggattgca agtggagaag tacgatacgt tatgataaga acaactcaga aaagctatag 660
   gttaagatcc tttcgcccat taactaagca gacattgtgg ttccctgcac agactccatg 720
   ctgtcaacat ggaaaatctc aactcaacaa gagcccagct tcccgtgtca gggatttctg 780
   gtgcttctca agctgtggct tcatcttatt gcccaactgt gacattcttt gattggaagg 840
   ggaaaactaa agcttttagc aaaaatacag ctagggaatt tgtcgatctg cgagagtaag 900
   acctcttatg atcctaacgg aatgatgtaa gctggaaata ataagcataa gatgaaattg 960
   aaaattgaag tctttattct ttaagaaaaa ctttgtactt gaaagcatgt ctgaagagtt 1020
   tactcattac cacaaacatc tagcatattg ataactaaca tctttatact ctacaagaga 1080
   ggctttccag ataggtacag tttttcttct ctattaggtc tatcaaaatt taacctatta 1140
   tgagggtcac ccctggcttt cactggtttt ctaaagaggc aagggtgtag taagaagcag 1200
   gcttaagttg ccttcctccc aatgtcaagt tcctttataa gctaatagtt taatcttgtg 1260
   aagatggcaa tgaaagcctg tggaagtgca aacctcacta tcttctggag ccaagtagaa 1320
   ttttcaagtt tgtagctctc acctcaagtg gttatgggtg tcctgtgatg aatctgctag 1380
   ctccagcctc agtctcctct cccacatcct ttcctttctt tcctctttga aacttctaag 1440
   aaaaagcaat ccaaacaagt tcagcactta agacacattg catgcacact tttgataagt 1500
   taaatccaac catctattta aaatcaaaat caggagatga gccaagagac cagaggttct 1560
   gttccagttt taaacagact tttactgaac atcccaatct tttaaccaca gaggctaaat 1620
   tgagcaaata gttttgccat ttgatataat ttccaacagt atgtttcaat gtcaagttaa 1680
   aaagtctaca aagctatttt ccctggagtg gtatcatcgc tttgagaatt tcttatggtt 1740
   aaaatggatc tgagatccaa gcatggcctg ggggatggtt ttgatctaag gaaaaaggtg 1800
   tctgtacctc acagtgcctt taaaacaagc agagatcccg tgtaccgccc taagatagca 1860
   cagactagtg ttaactgatt cccagaaaag tgtcacaatc agaaccaacg cattctctta 1920
   aactttaaaa atatgtattg caaagaactt gtgtacctgt aaatgtgtga ctgttgatga 1980
   cattatacac acatagccca cgtaagtgtc caatggtgct agcattggtt gctgagtttg 2040
   ctgctcgaaa gctgaagcag agatgcagtc cttcacaaag caatgatgga cagagagggg 2100
   agtctccatg ttttattctt ttgttgtttc tggctgtgta actgttgact tcttgacatt 2160
   gtgattttta tatttaagac aatgtattta ttttggtgtg tttattgttc tagcctttta 2220
   aatcactgac aatttctaat caagaagtac aaataattca atgcagcaca ggctaagagc 2280
   ttgtatcgtt tggaaaagcc agtgaaggct tctccactag ccatgggaaa gctacgcttt    2340
   agagtaaact agacaaaatt gcacagcagt cttgaacctc tctgtgctca agactcagcc    2400
   agtcctttga cattattgtt cactgtgggt gggaacacat tggacctgac acactgttgt    2460
   gtgtccatga aggttgccac tggtgtaagc tttttttggt tttcattctc ttatctgtag    2520
   aacaagaatg tggggctttc ctaagtctat tctgtatttt attctgaact tcgtatgtct    2580
   gagttttaat gttttgagta ctcttacagg aacacctgac cacacttttg agttaaattt    2640
   tatcccaagt gtgatattta gttgttcaaa aagggaaggg atatacatac atacatacat    2700
   acatacatac atatatatat atatatatac atatatatat atatatatat gtatatatat    2760
   atatatatag agagagagag agagagagag agagaaagag agagaggttg ttgtaggtca    2820
   taggagttca gaggaaatca gttatggccg ttaatactgt agctgaaagt gttttctttg    2880
   tgaataaatt catagcatta ttgatctatg ttattgctct gttttattta cagtcacacc    2940
   tgagaattta gttttaatat gaatgatgta ctttataact taatgattat ttattatgta    3000
   tttggttttg aatgtttgtg ttcatggctt cttatttaag acctgatcat attaaatgct    3060
   cccagtccgg a                                                          3071

## Description

### FIELD OF THE INVENTION

The present invention relates to a method for treating and preventing cancer/malignancies in mammals. More particularly, the present invention is directed to treating cancer by administering an effective amount of interleukin-21 (IL-21) to a subject, preferably human, in need thereof, such that the effective amount ameliorates, reduces, or eliminates the cancer.

### BACKGROUND OF THE INVENTION

Cytokines are a family of protein mediators of both natural and acquired immunity. They are extracellular proteins that modify the behavior of cells, particularly those cells that are in the immediate area of cytokine synthesis and release. In particular, cytokines are important in regulating hematopoiesis and immune responses. More specifically, cytokines mediate their actions through signal transduction. Accordingly, most cytokines bind to cells and transduce signals through either of the class I or class II cytokine receptors. The class I cytokine receptor family includes, but is not limited to, the receptors for interleukin (IL)-2, IL-3, IL-4, IL-5, IL-6, IL-7, IL-9, IL-11, IL-12, IL-13, and IL-15, as well as hematopoietic growth factors, leptin and growth hormones, while class II cytokine receptors include the receptors for IL-10 and the interferons (Cosman, Cytokines 5: 95-106, 1993).

A cytokine most closely related to IL-2 and IL-15 has been identified and is designated IL-21, and its class I receptor is designated IL-21R. Parrish-Novak et al. suggest that IL-21 plays a role in the proliferation and maturation of natural killer cells from bone marrow, in the proliferation of mature B cells co-stimulated with anti-CD40, and in the proliferation of T cells co-stimulated with anti-CD3 (Parrish-Novak et al., Nature 408: 57-63,2000). Sequencing of the full-length clone, IL-21R, demonstrated that this cDNA contained an open reading frame encoding a 538 amino acid protein having structural motifs common to class I cytokine receptors (Cosman, *supra;* Bazan, Proc. Natl. Acad Sci., USA 87: 6934-6938, 1990). Extracellular motifs include a single cytokine recognition module, two pairs of conserved cysteine residues, and a 'WSXWS' motif. The intracellular domain contains strong intracellular signaling motifs, including classical box 1 and box 2 motifs (Murkami et al. Proc. Natl. Acad Sci., USA 88: 11349-11353, 1951; Drachman and Kaushansky, Proc. Natl. Acad. Sci., USA 94: 2350-2355, 1997; Gurney, et al. Proc. Natl. Acad Sci., USA 92: 5292-5296, 1995), which indicate that the receptor can be a signaling subunit. IL-21R (GenBank Accession numbers AF254067 (human IL-21 R) and AF254068 (mouse IL-21R)) was shown to have the highest amino acid sequence similarity to IL-2R and IL-4Rα. Subsequently, Parrish-Novak et al. cloned mouse IL-21R from a mouse splenocyte library, and found that it shares overall structural and functional motifs with human IL-21R (Parrish-Novak et al., *supra*). Further, Parrish-Novak et al. describe the potent effects of IL-21 on all classes of lymphocytes: B, T, and natural killer cells (Parrish-Novak et al., *supra*). Additionally, Ozaki et al. found IL-21R abundantly expressed in lymphoid tissues, where expression occurs via the T cell antigen receptor, suggesting that the immune system can play a role (Proc. Natl. Acad Sci., USA 97:11439-11444, 2000).

Several cytokines known to mediate many of the immune responses involved in antitumor activity have been produced by recombinant DNA methodology and evaluated for their antitumor effects. In clinical trials, the administration of cytokines has resulted in objective tumor responses in patients with various types of neoplasms. More specifically, IL-2, an important cytokine in the generation of antitumor immunity that is structurally related to IL-21, can act locally at the site of tumor antigen stimulation to activate cytotoxic T-cells (CTL) and natural killer cells (NK), cellular immune activity which can mediate systemic tumor cell destruction.

Intravenous, intralymphatic, or intralesional administration of IL-2 has resulted in clinically significant responses in some cancer patients. However, severe toxicities (e.g., hypotension, pulmonary edema, prerenal azotemia, cardiac arrhythmias and myocardial infarction) limit the dose and efficacy of systemic IL-2 administration. The toxicity of systemically administered cytokines is not surprising, since these agents mediate local cellular interactions and they are normally secreted in limited quantities in a paracrine fashion.

Despite advances in cancer research, new treatments for cancer are needed. Novel immunotherapeutic approaches have been devised utilizing cytokines, such as IL-2 and interferon -a (IFN-α), or cell therapy. However, the toxicity of many of these agents, such as IL-2, is significant. Further, many patients do not respond well to currently available immunotherapeutic and chemotherapeutic agents.

### SUMMARY OF THE INVENTION

The present invention relates to the following embodiments as defined in items 1 to 11:
1. Use of an IL-21 polypeptide as shown in SEQ ID NO: 5 or 7 or a fragment thereof, wherein the fragment has the biological activity of IL-21, in the preparation of a medicament for treating or preventing cancer in a mammal.
2. Use of an IL-21 polynucleotide as shown in SEQ ID NO: 6 or 8 or a fragment thereof having the biological activity of IL-21 in the preparation of a medicament for treating or preventing cancer in mammal.
3. Use of an expression vector containing an IL-21 polynucleotide as shown in SEQ ID NO: 6 or 8 or a fragment thereof having the biological activity of IL-21 in the preparation of a medicament for treating or preventing cancer in a mammal.
4. The use according to item 3, wherein the expression vector is pORF.
5. The use according to any of items 1-4, wherein the cancer is a melanoma, a sarcoma, or a colon cancer.
6. The use according to item 1, wherein the IL-21 polypeptideor fragment thereof_is used together with a vaccine, an antigen-specific T lymphocyte, a cytokine, or a combination thereof in the preparation of the medicament.
7. The use according to item 2, wherein the IL-21 polynucleotide or fragment thereof is used together with a vaccine, an antigen-specific T lymphocyte, a cytokine, or a combination thereof in the preparation of the medicament.
8. The use according to item 3, wherein the expression vector is used together with a vaccine, an antigen-specific T lymphocyte, a cytokine, or a combination thereof in the preparation of the medicament.
9. The use according to any of items 6-8, wherein the vaccine is a recombinant viral vaccine or a peptide vaccine.
10. The use according to any of items 6-8, wherein the cytokine is IL-2, IL-7, or IL-15.
11. The use according to any of items 6-8, wherein the antigen-specific T lymphocyte is a tumor specific T lymphocyte.

Disclosed herein are methods of treating or preventing malignancies/cancer by administering to a patient in need thereof an effective therapeutic amount of an anti-cancer agent to prevent, treat or ameliorate the symptoms of the malignancies/cancers, where the anti-cancer agent is IL-21. Examples of malignancies/cancers include, for example, melanomas, lymphomas, sarcomas, colon cancer, and the like.

Further disclosed herein is a method of treating or preventing cancer in a subject, preferably human, comprising administering an IL-21 protein, polypeptide, or variant, alone or in combination with a carrier, buffer or saline, in an amount effective to the subject, to treat cancer by ameliorating, reducing, and eliminating cancer.

Further disclosed herein is a method for treating or preventing cancer in a subject, preferably human, comprising administering a DNA plasmid alone or in combination with a carrier, buffer or saline, to the subject, wherein the plasmid comprises the full-length IL-21 cDNA, and the plasmid and carrier are administered in an effective amount such that uptake of the plasmid occurs, and sufficient expression and secretion of the IL-21 protein results, to treat cancer by ameliorating, reducing, and eliminating cancer.

Further disclosed herein is a method for treating or preventing cancer in a subject by administering an effective amount of an IL-21 polypeptide, polynucleotide, vector encoding an IL-21 protein, variant, or fragment thereof, in combination with an immunotherapeutic and/or chemotherapeutic agent for the treatment and/or prevention of cancer.

### BRIEF DESCRIPTION OF THE DRAWINGS

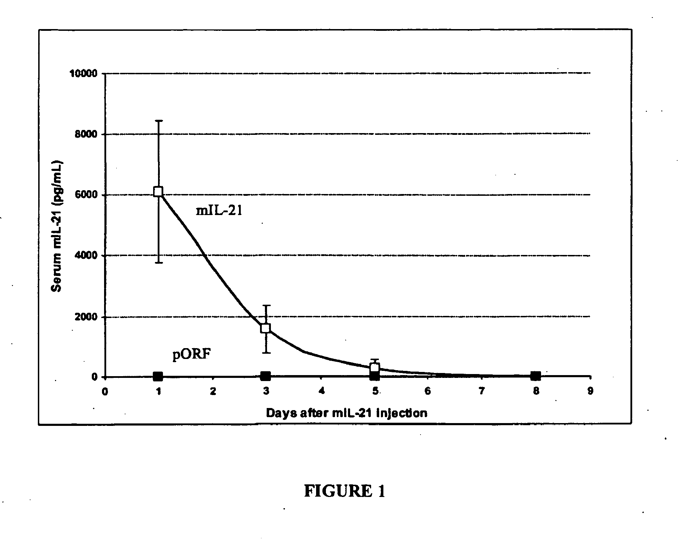

Figure 1 represents the expression of mIL-21 in mouse serum following DNA injection. C57BL/6 mice were intravenously injected with 20 µg of either mIL-21 or pORF control plasmid DNA in 2 mL of saline on day 0 as described in the Examples). On days 1, 3, 5 and 8, mice were sacrificed and serum samples were collected for mIL-21 ELISA analysis. Data represent one of 2 similar experiments. Each point represents data from 3 mice. Error bars are SEM.

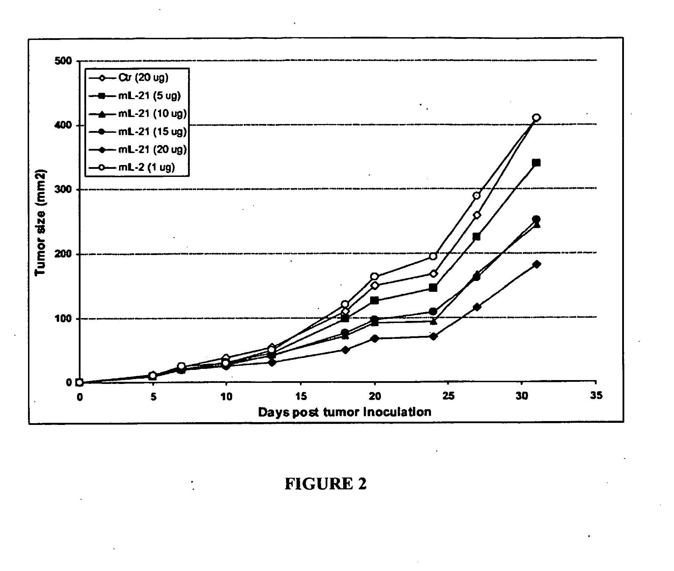

Figure 2 represents the dose response of mIL-21 in the treatment of MCA205 tumor *in vivo.* C57BL/6 mice were subcutaneously inoculated with 5x10⁵ MCA205 tumor cells on day 0. Five days later, tumor-bearing mice were intravenously injected with various doses of mIL-21 plasmid DNA ranging from 5 to 20 µg/mouse. Control mice were injected with either 20 µg of pORF or 1 µg of mIL-2 plasmid DNA. Injections were repeated 7 days later. Each group consisted of 5 mice.

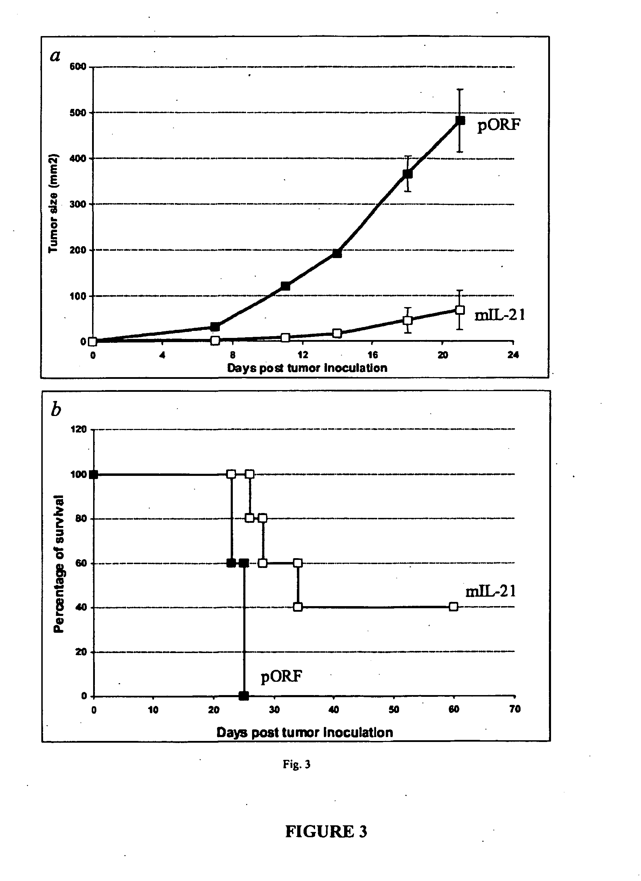

Figure 3 demonstrates that injection of mIL-21 plasmid DNA significantly inhibits B16 tumor growth *in vivo* and increases survival rate of tumor-bearing mice. Figure 3A represents the inhibition of B16 tumor growth *in vivo.* Figure 3B represents survival of B 16 tumor-bearing mice after mIL-2 treatment C57BI/6 mice were subcutaneously inoculated with 5x10⁵ B16 tumor on day 0, and treated with either 20 µg of mIL-21 DNA or pORF control DNA on days 5 and 12. Tumor growth and mouse survival rate were recorded. Data represent 1 of 3 experiments with similar results. Each group consisted of 5 mice. Differences between control and treatment groups in *3a* and *3b* are highly significant (p = 0.0001 and p = 0.0031, respectively).

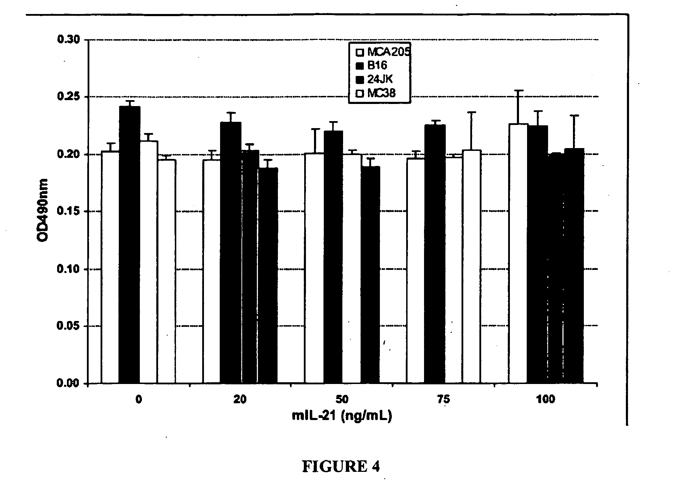

Figure 4 demonstrates that murine IL-21 does not inhibit tumor growth *in vitro.* 1x 10⁵ tumor cells/well were plated to 24 well plates, and various amounts of recombinant mIL-21 protein at the indicated concentrations were added to each well and incubated for 3 days. The culture medium from each well was then collected for an MTS-based cell proliferation assay. Data represent 1 of 3 independent experiments with similar results. The columns are the mean values of triplicates in each condition with standard deviations.

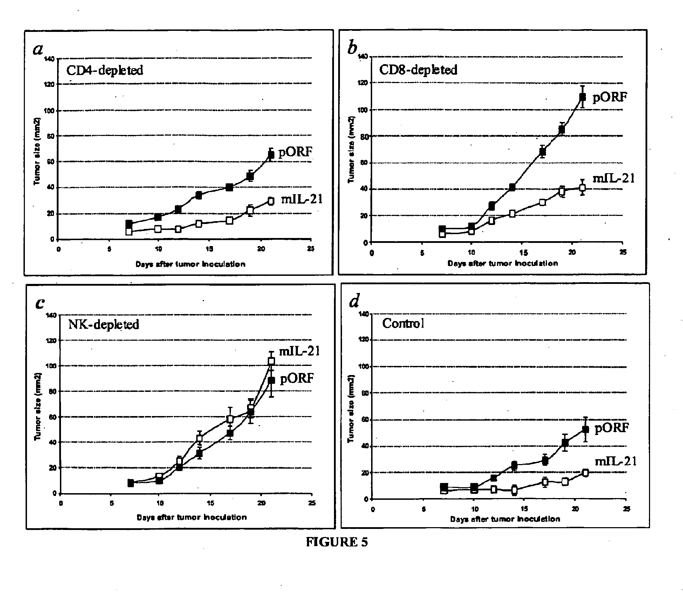

Figure 5 represents the antitumor effect of mIL-21 in CD4, CD8 and NK cell depleted mice. C57BL/6 mice were subcutaneously inoculated with 5x10⁵ MCA205 tumor cells on day 0. Antibodies against CD4, CDS or NK cells were administered on days 2 and 4, respectively. The depletion was maintained by repeated injection of antibodies every 6 to 7 days thereafter throughout the entire experiment. Treatment of mIL-21 began on day 5 and was repeated once 1 week later. Figure 5A represents CD4-depleted mice; Figure 5B represents CD8-depleted mice; Figure 5C represent NK-depleted mice; and Figure 5D represents control mice. Six mice were in each group. Data represent 1 of 3 similarly executed experiments with similar experiments. Error bars are SEM.

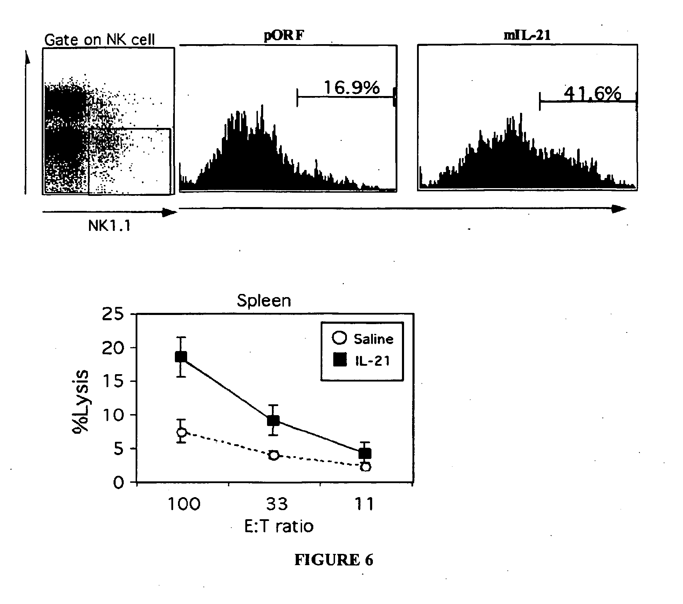

Figure 6 demonstrates that murine IL-21 enhances NK cell apoptosis and cytolytic activity *in vivo.* Figure 6A represents freshly isolated splenocytes from mice 4 days after either pORF or mIL-21 plasmid injection were stained with annexin V gated on NK1.1⁺/CD3" NK cells to detect apoptosis. Figure 6B represents the same splenocytes that were incubated with ⁵¹Cr-labeled YAC-1 target cells to determine the cytolytic activity of NK cells. Data represent results of 5 independent experiments with 3 mice in each group.

### DETAILED DESCRIPTION OF THE INVENTION

Disclosed herein are methods of treating a cancer in a mammal. In one method, the method comprises administering to a mammal afflicted with cancer an IL-21 polypeptide, variant, or fragment of either of the foregoing, in an amount effective to treat the cancer in the mammal. The method comprises administering to a mammal afflicted with cancer an IL-21 polynucleotide or fragment thereof, in an amount effective to treat the cancer in the mammal. The IL-21 polynucleotide or fragment thereof can be in the form of an expression vector, such that the method comprises administering to a mammal afflicted with cancer an expression vector containing an IL-21 polynucleotide or a fragment thereof in an amount effective to treat the cancer in the mammal.

Further disclosed herein are methods of treating an immune-related disease in a mammal. In one method, the method comprises administering to a mammal afflicted with an immune-related disease an IL-21 polypeptide, variant, or fragment of either of the foregoing, in an amount effective to treat the immune-related disease in the mammal. In another method, the method comprises administering to a mammal afflicted with an immune-related disease an I-21 polynucleotide or fragment thereof, in an amount effective to treat the immune-related disease in the mammal. The IL-21 polynucleotide or fragment thereof can be in the form of an expression vector, such that the method comprises administering to a mammal afflicted with an immune-related disease an expression vector containing an IL-21 polynucleotide in an amount effective to treat the immune-related disease in the mammal.

Methods of preventing a cancer in a mammal are also disclosed herein. In one method, the method comprises administering to a mammal an IL-21 polypeptide, variant, or fragment of either of the foregoing, in an amount effective to prevent the cancer in the mammal. In another method, the method comprises administering to a mammal an IL-21 polynucleotide or fragment thereof, in an amount effective to prevent the cancer in the mammal. The IL-21 polynucleotide or fragment thereof can be in the form of an expression vector, such that the method comprises administering to a mammal an expression vector containing an IL-21 polynucleotide or a fragment thereof in an amount effective to prevent the cancer in the mammal.

Further disclosed herein is a pharmaceutical composition comprising an IL-21 polypeptide, variant thereof, or fragment of either of the foregoing, and a pharmaceutically acceptable carrier, diluent, or excipient. Further provided is a pharmaceutical composition comprising an IL-21 nucleic acid molecule, or fragment thereof, and a pharmaceutically acceptable carrier, diluent, or excipient.

IL-21 is a cytokine produced by CD4⁺ T cells that is structurally related to IL-2, IL-4, and IL-15 (Parrish-Novak et al., Nature 408, 57-63 (2000)) and is known to have potent effects on all classes of lymphocytes, including B, T and NK cells. It acts synergistically on T cells with a proliferative signal provided by anti-CD3 antibodies, and promotes expansion of mature B cells in response to stimulation through CD40. In addition, IL-21, in synergy with Flt3 ligand and IL-15, promotes expansion and differentiation ofNK cells from bone marrow progenitors *in vitro*, and enhances lytic effector function against target cells in lysis assays. (Parrish-Novak et al. (2000), *supra*). The amino acid and nucleotide sequences of human IL-21 are known in the art and are publicly available at the National Center for Biotechnology Information (NCBI) website as GenBank Accession Nos. AAG29348 (SEQ ID NO:6) and AF254069 (SEQ ID NO:5), respectively. Furthermore, the amino acid and nucleotide sequences of mouse IL-21 are known in the art and are publicly available as GenBank Accession Nos. AAG29349 (SEQ ID NO:8) and AF254070 (SEQ ID NO:7) respectively.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

"Nucleic acid molecule" or "polynucleotid, as used herein, refers to any oligonucleotide or nucleotide sequence, fragments or portions of either of the foregoing, which encode all or part of IL-21. The nucleic acid molecule or polynucleotide can be DNA or RNA of either genomic or synthetic origin, which can be single- or double-stranded, and can be a coding (sense) or non-coding (anti-sense) strand. By way of nonlimiting example, fragments can be nucleic acid sequences that are greater than 10-60 nucleotides in length, and preferably include fragments that are at least 61-100 nucleotides, or which are 101 nucleotides or greater in length.

As used herein, an "IL-21 polynucleotide" refers to any nucleic acid sequence encoding an IL-21 molecule. For example, the IL-21 polynucleotide can contain the nucleotide sequence of the full-length IL-21 cDNA sequence. The IL-21 polynucleotide can also contain intronic sequences, 5' and/or 3' untranslated sequences, the coding region, the signal sequence, the secreted protein coding region, or any combination of the foregoing. Fragments, epitopes, domains, degenerate sequences, and variants of the IL-21 polynucleotide are also suitable for the present inventive methods and pharmaceutical composition.

The IL-21 polynucleotide can be composed of any polyribonucleotide or polydeoxyribonucleotide, which can be unmodified RNA or DNA or modified RNA or DNA. The IL-21 polynucleotide can contain one or more modified bases or modified intemucleotide linkages, wherein the modifications increase the stability of the polynucleotide or improve the polynucleotides in some other manner. "Modified" bases include for example, tritylated bases and unusual bases, such as inosine. A variety of modifications can be made to DNA and RNA; thus, "polynucleotide" embraces chemically, enzymatically, or metabolically modified forms.

Similarly, "an IL-21 amino acid sequence" or "IL-21 polypeptide" as used herein refers to an IL-21 oligopeptide, peptide, polypeptide, or protein sequence, and fragments or portions thereof, that are naturally occurring or are synthetic. Amino acid sequence fragments or portions thereof can be from about 5 to about 30 amino acids, preferably from: about 5 to about 15 amino acids in length. Such fragments and portions desirably retain the biological activity or function of the IL-21 polypeptide.

The terms "amino acid sequence" is recited herein to refer to an amino acid sequence of a naturally occurring "protein molecule, " amino acid sequence and like terms, such as "polypeptide" or "protein, " as used herein, are not meant to limit the amino acid sequence to the complete, native amino acid sequence associated with the recited protein molecule. Such molecules can also include fusion proteins, chimeric proteins, or other related proteins containing the functional portions of IL-21. The phrase "functional portions of IL-21" as used herein refers to any portion of IL-21 that has IL-21 biological activity. In addition, the terms "IL-21 polypeptide" and "IL-21 protein" are used interchangeably herein to refer to the encoded product of the IL-21 nucleic acid sequence of the present invention.

Moreover, as used herein, an IL-21 "polypeptide" refers to a molecule having the translated amino acid sequence generated from the IL-21 polynucleotide as broadly defined. "Secreted" IL-21 protein refers to a protein capable of being directed to the endoplasmic reticulum (ER), secretory vesicles, or the extracellular space as a result of a signal sequence, as well as an IL-21 protein released into the extracellular space. If the IL-21 secreted protein is released into the extracellular space, the IL-21 secreted protein can undergo extracellular processing to produce a "mature" IL-21 protein. Release into the extracellular space can occur by many mechanisms, including exocytosis and proteolytic cleavage.

A "variant" of the IL-21 polypeptide refers to an amino acid sequence that is altered by one or more amino acids. The variant can have "conservative" changes, wherein a substituted amino acid has similar structural or chemical properties, e.g., replacement of leucine with isoleucine. More rarely, a variant can have "non-conservative" changes, such as, for example, replacement of a glycine with a tryptophan. Minor variations can also include amino acid deletions or insertions, or both. Guidance in determining which amino acid residues can be substituted, inserted, or deleted without abolishing functional, biological or immunological activity can be found using computer programs well known in the art, for example, DNASTAR software. For purposes of the present invention, the variant preferably has an amino acid sequence that is at least 50% identical to the amino acid sequence of IL-21. More preferably, the variant has an amino acid sequence that is at least 85% identical to the amino acid sequence of IL-21. Most preferably, the variant has an amino acid sequence that is greater than 95% identical to the amino acid sequence of IL-21.

The IL-21 polypeptide can have one or more modifications, such as, but not limited to, glycosylation, acetylation, acylation, ADP-ribosylation, methylation, phosphorylation, carboxylation, esterification, myristoylation, and amidation (Proteins - Structure and Molecular Properties, 2nd Ed., Creighton, W. H. Freeman and Company, New York (1993); Posttranslational Covalent Modification Of Proteins, B. C. Johnson, Ed., Academic Press, New York, pp. 1-12 (1983)).

"Altered" nucleic acid sequences encoding IL-21 polypeptide include nucleic acid sequences containing deletions, insertions and/or substitutions of different nucleotides resulting in a polynucleotide that encodes the same or a functionally equivalent IL-21 polypeptide. Altered nucleic acid sequences can further include polymorphisms of the polynucleotide encoding the IL-21 polypeptide; such polymorphisms can or can not be readily detectable using a particular oligonucleotide probe. The encoded protein can also contain deletions, insertions, or substitutions of amino acid residues, which produce a silent change and result in a functionally equivalent IL-21 protein. Deliberate amino acid substitutions can be made on the basis of similarity in polarity, charge, solubility, hydrophobicity, hydrophilicity, and/or the amphipathic nature of the residues, as long as the biological activity of IL-21 protein is retained. For example, negatively charged amino acids can include aspartic acid and glutamic acid; positively charged amino acids can include lysine and arginine; and amino acids with uncharged polar head groups having similar hydrophilicity values can include leucine, isoleucine, and valine; glycine and alanine; asparagine and glutamine; serine and threonine; and phenylalanine and tyrosine.

"Peptide nucleic acid" (PNA) refers to an antisense molecule or anti-gene agent which comprises an oligonucleotide ("oligo") linked via an amide bond, similar to the peptide backbone of amino acid residues. PNAs typically comprise oligos of at least 5 nucleotides linked via amide bonds. PNAs can or can not terminate in positively charged amino acid residues to enhance binding affinities to DNA. Such amino acids include, for example, lysine and arginine, among others. These small molecules stop transcript elongation by binding to their complementary strand of nucleic acid (Nielsen et al., 1993, Anticancer Drug Des., 8: 53-63). PNAs can be pegylated to extend their lifespan in the cell where they preferentially bind to complementary single-stranded DNA and RNA.

An IL-21 polypeptide "having biological activity" refers to polypeptides exhibiting activity similar, but not necessarily identical to, an activity of an IL-21 polypeptide, including mature forms, as measured in a particular biological assay, with or without dose-dependency. In the case where dose-dependency does exist, it need not be identical to that of the IL-21 polypeptide, but rather substantially similar to the dose-dependence in a given activity as compared to the IL-21 polypeptide (i.e., the candidate polypeptide will exhibit greater activity or not more than about 25-fold less and, preferably, not more than about ten-fold less activity, and most preferably, not more than about threefold less activity relative to the IL-21 polypeptide). The biological activity of IL-21 is described in references, such as Parrish-Novak et al., (2000), *supra.*

A variety of techniques used to synthesize the IL-21 nucleic acid molecules of polynucleotides, are known in the art. See, for example Sambrook et al., 1989, *supra;* and Lemaitre et al., Proc. Nat'l. Acad. Sci., USA, 84: 648-652 (1987). The nucleic acid molecules or polynucleotides can alternatively be synthesized commercially by companies, such as Eurgentec, Belgium.

Methods of synthesizing IL-21 polypeptides, variants thereof, or fragments of either of the foregoing are also known in the art. The polypeptide (including the variants or fragments) can be synthesized using standard peptide synthesizing techniques well-known to those of skill in the art (e.g., as summarized in Bodanszky, Principles of Peptide Synthesis. Springer-Verlag, Heidelberg: 1984. In particular, the polypeptide can be synthesized using the procedure of solid-phase synthesis (see, e.g., Merrifield, J. Am. Chem. Soc., 85: 2149-54 (1963); Barany et al., Int. J. Peptide Protein Res., 30: 705-739 (1987); and U.S. Patent No. 5,424,398). If desired, this can be done using an automated peptide synthesizer. Removal of the t-butyloxycarbonyl (t-BOC) or 9-fluorenylmethyloxycarbonyl (Fmoc) amino acid blocking groups and separation of the polypeptide from the resin can be accomplished by, for example, acid treatment at reduced temperature. The polypeptide-containing mixture can then be extracted, for instance, with dimethyl ether, to remove non-peptidic organic compounds, and the synthesized polypeptide can be extracted from the resin powder (e.g, with about 25% w/v acetic acid). Following the synthesis of the polypeptide, further purification (e.g., using high performance liquid chromatography (HPLC)) optionally can be done in order to eliminate any incomplete polypeptides or free amino acids. Amino acid and/or HPLC analysis can be performed on the synthesized polypeptide to validate its identity. For other applications according to the invention, it can be preferable to produce the polypeptide as part of a larger fusion protein, either by chemical conjugation, or through genetic means, such as are known to those skilled in the art.

Further disclosed herein is a method of treating or preventing a cancer, pre-cancer, or immune-related disease, disorder, or condition in a subject. The subject can be any subject, but, preferably, the subject is a mammal. For purposes herein, mammals include, but are not limited to, dogs, cats, cows, horses, rabbits, monkeys, and humans. Most preferably, the mammal is a human. The method comprises administering an effective amount of an IL-21 protein, polypeptide, or variant or fragment thereof, such that the cancer, precancer, or immune-related disease, disorder, or condition is reduced, ameliorated, or eliminated. As used herein, the terms "reduce, " "ameliorate, " and "eliminate, " and words stemming therefrom, as used herein, do not necessarily imply a complete reduction, amelioration, or elimination. Rather, there are varying degrees of reduction, amelioration, and elimination of which one of ordinary skill in the art recognizes as having a potential benefit or prophylactic/therapeutic effect. In this regard, the reduction, amelioration, or elimination can be any level achieved through the present inventive methods. As used herein, the terms "treat" and "prevent, " and words stemming therefrom, as used herein, do not necessarily imply complete treatment or prevention. Rather, there are varying degrees of treatment and prevention of which one of ordinary skill in the art recognizes as having a potential benefit or prophylactic/therapeutic effect. In this regard, the treatment or prevention can be any level achieved through the present inventive methods. The IL-21 protein, polypeptide, or variant or fragment thereof, can be in the form of a derivative in which other constituents are attached thereto, such as, but not limited to, those forms that are labeled with a radioisotope, a biotin tag, or a fluorescein tag. A targeting agent also may be used to allow for specific targeting to a specific organ, tumor, or cell types. Such targeting agents can be hormones, immunoglobulins, cytokines, cellular receptors and the like. The IL-21 protein, peptide, or variant thereof, can be administered alone, or in combination with other reagents or therapeutics.

Further disclosed herein is a method of treating or preventing a cancer, precancer, or immune-related disease, disorder, or condition in a subject having a cancer, precancer, or immune-related disease, by administering a pharmaceutical composition in which an IL-21 protein or polypeptide is formulated with a pharmaceutically acceptable carrier by methods known in the art.

Further disclosed herein is a method of treating a subject, preferably a mammalian subject, more preferably a human subject, having a solid tumor or lymphoma, by administering an effective amount of an IL-21 protein, polypeptide, or variant or fragment thereof, or a pharmaceutical composition containing an IL-21 protein, peptide or variant thereof, such that the tumor or lymphoma is reduced, ameliorated, or eliminated. Examples of other diseases, disorders, and/or conditions that can be treated include, but are not limited to, adenocarcinoma, leukemia, lymphoma, melanoma, myeloma, sarcoma, and teratocarcinoma, and particularly, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, colon, and uterus. Preferably, the cancer is melanoma, a sarcoma, or colon cancer.

Further disclosed herein is a method of treating or preventing a subject, having a cancer, precancer, or immune-related disease, disorder, or condition by administering an IL-21 nucleic acid molecule, polynucleotide, or vector comprising a polynucleotide encoding an IL-21 polypeptide, in an amount effective such that the cancer, precancer, or immune-related disease, disorder, or condition is reduced, ameliorated, or eliminated.

The present invention relates to the use of a novel anti-cancer agent for treating human cancers, neoplasms and/or malignancies. A plasmid suitable for IL-21 expression has been developed for use as an anti-cancer agent in the treatment of subjects with cancer, such as, but not limited to, solid tumors or lymphomas. One approach relies on the direct administration of recombinant genes into established tumor cells *in vivo*, to modify them genetically, as they grow *in situ,* to produce and secrete local amounts of IL-21. The secretion of local amounts of IL-21 by tumor cells can cause subsequent tumor reduction or eradication. In the present method, genes can be directly transferred into solid tumor sites, where local cells take up and express the gene. In some sites, such as skeletal and cardiac muscle, expressible DNA can be injected without using carriers. In other tissues, DNA expression can be facilitated by introducing the DNA complexed with a cationic lipid, such as, for example, in a lipid complex or liposome. The lipid component facilitates the entry of the DNA into those cells provided access to the DNA/lipid complex. Delivery of DNA to patients in a drug-like manner thereby may be facilitated.

Accordingly, one method of treating or preventing a subject having a cancer can be achieved by inserting a gene encoding IL-21 protein or peptides into high efficiency expression systems, such as *E coli,* yeast, baculovirus, vaccinia virus, and the like. Techniques using non-viable DNA vectors have the advantage of ease of preparation and safety of administration. The IL-21 nucleic acid sequence is, therefore, useful as an anti-cancer agent. The DNA sequences encoding the IL-21 proteins or peptides of the present invention can be administered using a gene gun in amounts to elicit a cellular response against a cancer cell. Nanogram quantities are useful for such purposes.

Further disclosed herein is a method of treating or preventing a subject, preferably mammalian, more preferably human, having solid malignant tumors or lymphomas by administering to the subject an effective amount of an IL-21 plasmid suitable to reduce, ameliorate, and/or eliminate the tumor or lymphoma. The IL-21 plasmid DNA can be directly introduced into the solid tumor cells or nodules of the patient. Further disclosed herein is

Further disclosed herein is a method of treating or preventing cancer, precancer, or an immune-related disease, disorder, or condition in a subject by administering an IL-21 polypeptide or variant thereof, polynucleotide, and/or vector comprising a polynucleotide encoding an IL-21 polypeptide, in combination with other appropriate therapeutic agents in an amount effective to reduce, ameliorate, or eliminate the cancer, precancer, or immune-related disease, disorder, or condition. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one can achieve therapeutic efficacy with lower dosages of each agent, thus reducing the potential for adverse side effects.

More specifically, when treating or preventing a solid tumor (either malignant or benign), by administering a plasmid comprising an IL-21 gene, the IL-21 plasmid is directly transferred into solid tumor sites, where local cells take up and express the gene. In some sites, such as, but not limited, to skeletal and cardiac muscle, expressible DNA can be injected without using carriers. In other tissues, such as tumor cells, DNA expression can be facilitated by introducing the DNA and carrier, for example, a lipid. The lipid component can facilitate the entry of the DNA into those cells provided access to the DNA/lipid complex. Delivery of the IL-21 DNA to patients in a drug-like manner is, thus, facilitated.

In particular, the direct gene transfer approach utilizes a plasmid suitable for IL-21 expression. A preferred plasmid is a circular, double-stranded DNA plasmid that is a simplified eukaryotic expression vector. The gene for IL-21 can be inserted into the plasmid so that IL-21 is expressed when the plasmid is introduced into cells. Other genes also can be included to aid and enhance expression of IL-21. In one embodiment, IL-21 can be inserted into pORF5-mcs (Invivogen), where the multiple cloning sites (mcs) include some of the following restriction sites: Sgr AI, Sal I, Bam HI, Pst I, Nco I, and Nhe I. Accordingly, IL-21 is placed under the transcriptional control of elongation factor 1 α/eukaryotic initiation factor 4g (EF-1α/eIF4g). Methods, which are well-known to those skilled in the art, can be used to construct expression vectors containing sequences encoding the IL-21 polypeptide and appropriate transcriptional and translational control elements. These methods include *in vitro* recombinant DNA techniques, synthetic techniques, and *in vivo* genetic recombination. Such techniques are described in J. Sambrook et al., 1989, Molecular Cloning, A Laboratory Manual, Cold Spring Harbor Press, Plainview, NY, and in Ausubel et al., 1989, Current Protocols in Molecular Biology, John Wiley & Sons, New York, NY.

A variety of expression vector or host systems can be utilized to contain and express sequences encoding the IL-21 polypeptide. Such expression vector/host systems include, but are not limited to, microorganisms, such as bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus (CaMV) and tobacco mosaic virus (TMV)), or with bacterial expression vectors (e.g., Ti or pBR322 plasmids); or animal cell systems. The host cell employed is not limiting to the present invention.

"Control elements" or "regulatory sequences" are those non-translated regions of the vector, e.g., enhancers, promoters, 5' and 3' untranslated regions, which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters, such as the hybrid lacZ promoter of the BLUESCRIPT phagemid (Stratagene; La Jolla, CA) or PSPORT1 plasmid (Life Technologies; Rockville, MD), and the like, can be used. The baculovirus polyhedrin promoter can be used in insect cells. Promoters or enhancers derived from the genomes of plant cells (e.g., heat shock, RUBISCO; and storage protein genes), or from plant viruses (e.g., viral promoters or leader sequences), can be cloned into the vector. In mammalian cell systems, promoters from mammalian genes or from mammalian viruses are preferred. If it is necessary to generate a cell line that contains multiple copies of the sequence encoding IL-21, vectors based on SV40 or EBV can be used with an appropriate selectable marker.

In bacterial systems, a number of expression vectors can be selected, depending upon the use intended for the expressed IL-21 product. For example, when large quantities of expressed protein are needed for the induction of antibodies, vectors which direct high-level expression of fusion proteins that are readily purified can be used. Such vectors include, but are not limited to, the multifunctional *E. coli* cloning and expression vectors, such as BLUESCRIPT (Stratagene; La Jolla, CA), in which the sequence encoding the IL-21 polypeptide can be ligated into the vector in-frame with sequences for the amino-terminal Met and the subsequent 7 residues of ß-galactosidase, so that a hybrid protein is produced; pIN vectors (see, Van Heeke and Schuster, 1989, J. Biol. Chem., 264:5503-5509); and the like. pGEX vectors (Promega; Madison, WI) also can be used to express foreign polypeptides, as fusion proteins with glutathione S-transferase (GST). In general, such fusion proteins are soluble and can be easily purified from lysed cells by adsorption to glutathione-agarose beads followed by elution in the presence of free glutathione. Proteins made in such systems can be designed to include heparin, thrombin, or factor XA protease cleavage sites so that the cloned polypeptide of interest can be released from the GST moiety at will.

In mammalian host cells, a number of viral-based expression systems can be utilized. In cases where an adenovirus is used as an expression vector, sequences encoding the IL-21 polypeptide can be ligated into an adenovirus transcription/ translation complex containing the late promoter and tripartite leader sequence. Insertion in a non-essential E1 or E3 region of the viral genome can be used to obtain a viable virus, which is capable of expressing the IL-21 polypeptide in infected host cells (Logan and Shenk, 1984, Proc. Natl. Acad. Sci., USA 81:3655-3659). In addition, transcription enhancers, such as the Rous sarcoma virus (RSV) enhancer, can be used to increase expression in mammalian host cells.

Specific initiation signals also can be used to achieve more efficient translation of sequences encoding the IL-21 polypeptide. Such signals include the ATG initiation codon and adjacent sequences. In cases where sequences encoding the IL-21 polypeptide, its initiation codon, and upstream sequences are inserted into the appropriate expression vector, no additional transcriptional or translational control signals can be needed. However, in cases where only the coding sequence, or a fragment thereof, is inserted, exogenous translational control signals, including the ATG initiation codon, can be provided. Furthermore, the initiation codon is preferably in the correct reading frame to ensure translation of the entire insert. Exogenous translational elements and initiation codons can be of various origins, both natural and synthetic. The efficiency of expression can be enhanced by the inclusion of enhancers, which are appropriate for the particular cell system that is used, such as those described in the literature (Scharf et al., 1994, Results Probl. Cell Differ., 20:125-162),

In one instance, the IL-21 polynucleotide and/or polypeptide, including agonists, antagonists, and fragments thereof, are useful for modulating signaling pathways. In one embodiment of the present invention, an expression vector containing the polynucleotide encoding the IL-21 polypeptide can be administered to an individual to treat or prevent a neoplastic disorder, including, but not limited to, the types of cancers and tumors described above.

Polypeptides used in treatment also can be generated endogenously in the subject, in treatment modalities often referred to as "gene therapy". Thus, for example, cells from a subject can be engineered with a polynucleotide, such as DNA or RNA, to encode a polypeptide *ex vivo,* and for example, by the use of a retroviral plasmid vector. The cells then can be introduced into the subject. Further details regarding gene therapy, and specifically on dosage and frequency of cells, are provided in U.S. Patent No. 5,399,346, which is incorporated herein by reference, *in toto.*

The genes encoding an IL-21 polypeptide can be turned on or off by transforming a cell or tissue with an expression vector that expresses high levels of an IL-21 polypeptide-encoding polynucleotide, or a fragment thereof or the complementary sequence thereof. Such constructs can be used to introduce untranslatable sense or antisense sequences into a cell. Even in the absence of integration into the DNA, such vectors can continue to transcribe RNA molecules until they are disabled by endogenous nucleases. Transient expression can last for a month or more with a non-replicating vector, and even longer if appropriate replication elements are designed to be part of the vector system.

Modifications of gene expression can be obtained by designing antisense molecules or complementary nucleic acid sequences (DNA, RNA, or PNA), to the control, 5', or regulatory regions of the gene encoding an IL-21 polypeptide, (e.g., signal sequence, promoters, enhancers, and introns). Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or regulatory molecules. Recent therapeutic advances using triplex DNA have been described (see, for example, Gee et al., 1994, In: Huber and Carr, Molecular and Immunologic Approaches, Futura Publishing Co., Mt. Kisco, NY). The antisense molecule or complementary sequence also can be designed to block translation of mRNA by preventing the transcript from binding to ribosomes.

Ribozymes, i.e., enzymatic RNA molecules, also can be used to catalyze the specific cleavage of RNA. The mechanism of ribozyme action involves sequence-specific hybridization of the ribozyme molecule to complementary target RNA, followed by endonucleolytic cleavage. Suitable examples include engineered hammerhead motif ribozyme molecules that can specifically and efficiently catalyze endonucleolytic cleavage of sequences encoding IL-21 polypeptide.

Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site can be evaluated for secondary structural features, which can render the oligonucleotide inoperable. The suitability of candidate targets also can be evaluated by testing accessibility to hybridization with complementary oligonucleotides using ribonuclease protection assays.

Complementary ribonucleic acid molecules and ribozymes according to the invention can be prepared by any method known in the art for the synthesis of nucleic acid molecules. Such methods include techniques for chemically synthesizing oligonucleotides, for example, solid phase phosphoramidite chemical synthesis. Alternatively, RNA molecules can be generated by *in vitro* and *in vivo* transcription of DNA sequences encoding IL-21. Such DNA sequences can be incorporated into a wide variety of vectors with suitable RNA polymerase promoters, such as T7 or SP. Alternatively, the cDNA constructs that constitutively or inducibly synthesize complementary RNA can be introduced into cell lines, cells, or tissues.

RNA molecules can be modified to increase intracellular stability and half-life. Possible modifications include, but are not limited to, the addition of flanking sequences at the 5' and/ or 3' ends of the molecule, or the use of phosphorothioate or 2' O-methyl, rather than phosphodiesterase linkages within the backbone of the molecule. This concept is inherent in the production of PNAs and can be extended in all of these molecules by the inclusion of nontraditional bases, such as inosine, queosine, and wybutosine, as well as acetyl-, methyl-, thio-, and similarly modified forms of adenine, cytosine, guanine, thymine, and uridine, which are not as easily recognized by endogenous endonucleases.

Many methods for introducing vectors into cells or tissues are available and are equally suitable for use *in vivo*, *in vitro*, and *ex vivo.* For *ex vivo* therapy, vectors can be introduced into stem cells taken from the patient and clonally propagated for autologous transplant back into that same patient. Delivery by transfection and by liposome injections can be achieved using methods, which are well-known in the art.

Further disclosed herein is a method of treating or preventing a cancer, precancer, or immune-related disease, disorder, or condition by administrating a pharmaceutical composition, in conjunction with a pharmaceutically acceptable carrier, diluent, or excipient, for any of the above-described therapeutic uses and effects. Such pharmaceutical compositions can comprise IL-21 nucleic acid, polypeptide, or peptides, activating antibodies to IL-21 receptor, mimetics, agonists, antagonists, or modulators of IL-21 polypeptide or polynucleotide. In a further instance, it can be useful to administer pharmaceutical compositions comprising neutralizing or inhibitory antibodies to IL-21 receptor for the treatment of autoimmune diseases or instances where inhibiting IL-21 receptor and ligand interactions have beneficial effects, as exemplified by blocking CD40-CD40L interactions (Diehl et al., J. Mol. Med. 78: 363-371, 2000; Datta and Kalled, Arthritis & Rheumatism 40: 1735-1745, 1997). The compositions can be administered alone, or in combination with at least one other agent, such as a stabilizing compound, which can be administered in any sterile, biocompatible pharmaceutical carrier, including, but not limited to, saline, buffered saline, dextrose, and water. The compositions can be administered to a patient alone, or in combination with other agents, drugs, hormones, or biological response modifiers.

In a one instance the proteins, complementary sequences, or vectors of the present invention can be administered alone, or in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one can be able to achieve therapeutic efficacy with lower dosages of each agent, thus reducing the potential for adverse side effects.

In this regard, the methods of treating cancer in a mammal and methods of preventing cancer can comprise co-administering other therapeutic or prophylactic agents, with an IL-21 polypeptide, variant, fragment of the foregoing, IL-21 polynucleotide or fragment thereof, or an expression vector containing an IL-21 polynucleotide or fragment thereof. Such agents include, for example, a vaccine, an antigen-specific T lymphocyte, and a cytokine. The vaccine can be a recombinant viral vaccine or a peptide vaccine. The antigen-specific T lymphocyte can be, for instance, a tumor-specific T lymphocyte. The cytokine can be any cytokine. Preferably, the cytokine is IL-2, Il-7, or IL-15. One of ordinary skill in the art recognizes that the present inventive methods of treating/preventing cancer include any combination of the methods described herein.

In particular, the combination approach relates to tumor cells that are collected, propagated *in vitro,* modified and selected and then reinjected *in vivo*. More specifically, this embodiment relates to passive immunotherapy with genetically modified immune cells (commonly referred to as adoptive immunotherapy) capable of recognizing human tumor antigens effective in mediating the regression of cancer in selected patients with metastatic melanoma. *In vitro* techniques have been developed in which human lymphocytes are sensitized *in vitro* to tumor antigen immunodominant peptides presented on antigen presenting cells. Administration of IL-21 polypeptide or polynucleotide can be used in conjunction to increase the effects of adoptive immunotherapy for the treatment and/or prevention of cancer in a subject

T cells from immunized mammals that have specific reactivity against cancer, precancer, or an immune-related disease, disorder, or condition, also can be used *in vivo* for the treatment of individuals afflicted with cancer by administering from about 10⁷ to 10¹¹ T cells to a mammal intravenously, intraperitoneally, intramuscularly, or subcutaneously in addition to IL-21 polypeptide or polynucleotide. Preferred routes of administration are intravenously or intraperitoneally.

For example, incorporation of the gene for IL-2 can increase the immunogenicity of tumor antigens and even mediate the regression of established lung metastases bearing these antigens and even mediate the regression of established lung metastases bearing these antigens. Active immunotherapy followed by the exogenous administration of co-immunostimulatory cytokines, such as IL-2, IL-6, IL-10, and, preferably, IL-21 also can be used to improve immune responses.

Further disclosed herein is a method for inducing an immunological response in a mammal comprising inoculating the mammal with IL-21 polypeptide, or a fragment thereof, adequate to produce antibody and/or T cell immune response to protect the mammal from infections, such as bacterial, fungal, protozoan and viral infections, or infections caused by HIV-1 or HIV-2, cancer, precancer, and other immune-related diseases, disorders, or conditions. Further disclosed herein is a method of inducing immunological response in a mammal comprising, delivering IL-21 polypeptide via a vector directing expression of IL-21 polynucleotide *in vivo* in order to induce such an immunological response to produce antibody to protect the mammal from the diseases, disorders, or conditions described above.

Further disclosed herein is an immunological or vaccine formulation or composition which, when introduced into a mammalian host, induces an immunological response in the mammal to an IL-21 polypeptide, where the composition comprises an IL-21 polypeptide or IL-21 gene. The formulation can further comprise a suitable carrier. Since the IL-21 polypeptide can be broken down in the stomach, it is preferably administered parenterally (including subcutaneous, intravenous, intramuscular, intradermal, etc., injection). Formulations suitable for parenteral administration include aqueous and non-aqueous sterile injection solutions, which can contain anti-oxidants, buffers, bacteriostats and solutes, which render the formulation isotonic with the blood of the recipient; and aqueous and non-aqueous sterile suspensions, which can include suspending agents or thickening agents. The formulations can be presented in unit-dose or multi-dose containers, for example, sealed ampoules and vials, and can be stored in a freeze-dried condition, requiring only the addition of the sterile liquid carrier immediately prior to use. The vaccine formulation also can include adjuvant systems for enhancing the immunogenicity of the formulation, such as oil-in-water systems and other systems known in the art. Furthermore, IL-21 also can be used as an adjuvant either alone or in combination with other reagents to enhance the immunization or vaccination against cancer, precancer, and/or immune-related diseases, disorders, or conditions. The dosage will depend on the specific activity of the vaccine and can be readily determined by routine experimentation.

Further disclosed herein is methods of preventing or inhibiting cancer, precancer, preferably tumors or lymphomas, and immune-related diseases, disorders, or conditions in mammals, by administering IL-21 protein or peptides (or nucleic acid sequences encoding them) to the mammal via routes of administration that include, but are not limited to intravenous, subcutaneous, intratumor, intramuscular, intradermal, intraperitoneal, intrathecal, intraplerural, intrauterine, rectal, vaginal, topical, and the like.

Administration also can be by transmucosal or transdermal means. For transmucosal or transdermal administration penetrants appropriate to the barrier to be permeated are used in the formulation. Such penetrants are generally known in the art, and include, for example, for transmucosal administration bile salts and fusidic acid derivatives. In addition, detergents can be used to facilitate permeation. Transmucosal administration can be by nasal sprays, for example, or suppositories. For oral administration, the IL-21 protein, peptides, or variants thereof are formulated into conventional oral administration forms, such as capsules and tablets.

In addition to administering IL-21 molecules alone, pharmaceutical compositions comprising a therapeutically effective amount of one or more IL-21 molecules in a mixture with a pharmaceutically acceptable carrier can be used. This composition can be administered either parenterally, intravenously or subcutaneously. When administered, the therapeutic composition for use in this invention is preferably in the form of a pyrogen-free, parenterally acceptable aqueous solution. The preparation of such a parenterally acceptable protein solution, having due regard to pH, isotonicity, stability and the like, is within the skill of the art.

The pharmaceutical compositions administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, vaginal, or rectal means.

In addition to the active ingredients (i.e., an IL-21 nucleic acid and/or polypeptide, and/or functional fragments thereof (Parrish-Novak et al., Nature 408: 57-63 (2000) which is hereby incorporated by reference *in toto*)), the pharmaceutical compositions can contain suitable pharmaceutically acceptable carriers or excipients comprising auxiliaries, which facilitate processing of the active compounds into preparations, which can be used pharmaceutically. Further details on techniques for formulation and administration are provided in the latest edition of Remington's Pharmaceutical Sciences (Mack Publishing Co., Easton, PA).

Pharmaceutical compositions for oral administration can be formulated using pharmaceutically acceptable carriers well-known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained by the combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropyl-methylcellulose, or sodium carboxymethylcellulose; gums, including arabic and tragacanth, and proteins such as gelatin and collagen. If desired, disintegrating or solubilizing agents can be added, such as cross-linked polyvinyl pyrrolidone, agar, alginic acid, or a physiologically acceptable salt thereof, such as sodium alginate.

Dragee cores can be used in conjunction with physiologically suitable coatings, such as concentrated sugar solutions, which can also contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification, or to characterize the quantity of active compound, i.e., dosage.

Pharmaceutical preparations, which can be used orally, include push-fit capsules made of gelatin, as well as soft, scaled capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances, which increase the viscosity of the suspension, such as sodium carboxymethyl cellulose, sorbitol, or dextran. In addition, suspensions of the active compounds can be prepared as appropriate oily injection suspensions. Suitable lipophilic solvents or vehicles include fatty oils such as sesame oil, or synthetic fatty acid esters, such as ethyloleate or triglycerides, or liposomes. Optionally, the suspension also can contain suitable stabilizers or agents which increase the solubility of the compounds to allow for the preparation of highly concentrated solutions.

For topical or nasal administration, penetrants or permeation agents that are appropriate to the particular barrier to be permeated are used in the formulation. Such penetrants are generally known in the art.

The pharmaceutical compositions of the present invention can be manufactured in a manner that is known in the art, e.g., by means of conventional mixing, dissolving, granulating, dragee-making, levigating, emulsifying, encapsulating, entrapping, or lyophilizing processes.

The pharmaceutical composition can be provided as a salt and can be formed with many acids, including but not limited to, hydrochloric, sulfuric, acetic, lactic, tartaric, malic, succinic, and the like. Salts tend to be more soluble in aqueous solvents, or other protonic solvents, than are the corresponding free base forms. In other cases, the preferred preparation can be a lyophilized powder, which can contain any or all of the following: 1-50 mM histidine, 0.1%-2% sucrose, and 2-7% mannitol, at a pH range of 4.5 to 5.5, combined with a buffer prior to use. After the pharmaceutical compositions have been prepared, they can be placed in an appropriate container and labeled for treatment of an indicated condition. For administration of IL-21 product, such labeling would include amount, frequency, and method of administration.

Pharmaceutical compositions suitable for use in the present invention include compositions in which the active ingredients are contained in an effective amount to achieve the intended purpose. The determination of an effective dose or amount is well within the capability of those skilled in the art. For any compound, the therapeutically effective dose can be estimated initially either in cell culture assays, e.g., using neoplastic cells, or in animal models, usually mice, rabbits, dogs, or pigs. The animal model also can be used to determine the appropriate concentration range and route of administration. Such information then can be used and extrapolated to determine useful doses and routes for administration in humans.

A therapeutically effective dose refers to that amount of active ingredient, for example, IL-21 polypeptide, or fragments thereof, which ameliorates, reduces, or eliminates the cancer. Therapeutic efficacy and toxicity can be determined by standard pharmaceutical procedures in cell cultures or experimental animals, e.g., ED₅₀ (the dose therapeutically effective in 50% of the population) and LD₅₀ (the dose lethal to 50% of the population). The dose ratio of toxic to therapeutic effects is the therapeutic index, which can be expressed as the ratio, LD₅₀/ED₅₀. Pharmaceutical compositions exhibiting large therapeutic indices are preferred. The data obtained from cell culture assays and animal studies are used in determining a range of dosages for human use. Preferred dosage contained in a pharmaceutical composition is within a range of circulating concentrations that include the ED₅₀ with little or no toxicity. The dosage varies within this range depending upon the dosage form employed, sensitivity of the patient, and the route of administration.

The practitioner, who will consider the factors related to the individual requiring treatment, will determine the exact dosage. Dosage and administration are adjusted to provide sufficient levels of the active moiety or to maintain the desired effect. Factors, which can be taken into account, include the severity of the individual's disease state, general health of the patient, age, weight, and gender of the patient, diet, time and frequency of administration, drug combination(s), reaction sensitivities, and tolerance/response to therapy. As a general guide, long-acting pharmaceutical compositions can be administered every 3 to 4 days, every week, or once every two weeks, depending on half-life and clearance rate of the particular formulation. Variations in these dosage levels can be adjusted using standard empirical routines for optimization, as is well understood in the art.

Normal dosage amounts can vary from 0.1 to 100,000 micrograms (µg), up to a total dose of about 1 gram (g), depending upon the route of administration. Guidance as to particular dosages and methods of delivery is provided in the literature and is generally available to practitioners in the art. Those skilled in the art will employ different formulations for nucleotides than for proteins or their inhibitors. Similarly, delivery of polynucleotides or polypeptides will be specific to particular cells, conditions, locations, and the like.

Generally, it is desirable to provide the recipient with a dosage of IL-21 protein or peptide of at least about 1 pg/ kg body weight, preferably at least 1 ng/ kg body weight, more preferably at least about 1 µg/ kg body weight or greater of the recipient. A range of from about 1 µg/ kg body weight to about 100 mg/ kg body weight is preferred, and a range from 10 µg/kg body weight to 10 mg/kg body weight is more preferred, although a lower or higher dose can be administered. The desired dose is effective to prevent or inhibit cancer, precancer, and immune-related diseases, disorders, or conditions in the recipient, preferably human.

The dosage regimen involved in a method for treating the above-described conditions will be determined by the attending physician considering various factors which modify the action of drugs, e.g. the condition, body weight, sex and diet of the patient, the severity of any infection, time of administration and other clinical factors. Generally, a daily regimen can be in the range of about 1 mg to about 2.5 mg of IL-21 plasmid DNA per kilogram of body weight. Dosages would be adjusted relative to the activity of, for example, IL-21 and it would not be unreasonable to note that dosage regimens can include doses as low as 1 microgram and as high as 5 milligram per kilogram of body weight per day. In addition, there can exist specific circumstances where dosages of IL-21 would be adjusted higher or lower than this range. For example, when IL-21 is used as an adjuvant, the dose can be much lower, such as 1 microgram per kilogram body weight or per injection site. These include co-administration with other anti-cancer agents and/or co-administration with chemotherapeutic drugs and/or radiation. As indicated above, the therapeutic method and compositions also can include co-administration with other human factors. A non-exclusive list of other appropriate hematopoietins, CSFs, cytokines, lymphokines, hematopoietic growth factors and interleukins for simultaneous or serial co-administration with the polypeptides of the present invention includes GM-CSF, CSF-1, G-CSF, Meg-CSF (more recently referred to as c-mpl ligand), M-CSF, erythropoietin (EPO), IL-1, IL-4, IL-2, IL-5, IL-6, IL-7, IL-8, IL-9, IL-10, IL-11, IL-12, IL-13, LIF, flt3 ligand, B-cell growth factor, B-cell differentiation factor and eosinophil differentiation factor, stem cell factor (SCF) also known as steel factor or c-kit ligand, or combinations thereof. The dosage recited above would be adjusted to compensate for such additional components in the therapeutic composition. Progress of the treated patient can be monitored by periodic assessment of the cancer profile, e.g., blood cell count and the like.

*

### EXAMPLES

*Cell lines and reagents:* B16 melanoma and MCA205 fibrosarcoma tumor lines were cultured in RPMI 1640 complete medium supplemented with 10 % heat-inactivated FBS, L-glutamine, sodium pyruvate, non-essential amino acids, and penicillin-streptomycin (all from Invitrogen/Life Technologies, Rockville, MD). Anti-mouse monoclonal antibodies against CD4 (GK1.5) and CDS (2.43) were obtained from NCI BRB Preclinical Repository (Frederick, MD). Anti-asialo GM1 antibody against mouse NK cells was purchased from Wako Pure Chemical Industries, Osaka, Japan. Recombinant murine IL-21 was purchased from R&D Systems (Minneapolis, MN). Antibodies used for FACS analysis were purchased from BD/PharMingen (San Diego, CA).

*Statistics:*

The statistical analyses to compare tumor growth rate and mouse survival rate between treatment and control groups were determined by ANOVA- Repeated Measures test using the StatView program (Abacus Concepts, Berkeley, CA). The statistical analyses to compare tumor sizes and cell numbers between treatment and control groups were determined by the nonparametric Kruskal-Wallis test using the StatView program.

Example 1

This example demonstrates the cloning of human and murine IL-21.

Human PBMC and murine spleen cells (C57BL/6) were activated by 5 ng/ml of PMA and 250 µg/ml Ionomycin for 24 hr. Total RNA was extracted and isolated by TRIZOL method (Life Technologies/Invitrogen; Carlsbad, CA). RT-PCR was performed to amplify the first strand of cDNA by random primers according to manufacturer's instruction (ThermoScript RT-PCR System; Life Technologies/ Invitrogen). The full-length cDNA fragment (including the original signal peptide) was PCR amplified using a pair of specific primers for either human or murine IL-21.

The human IL-21 primers used were as follows:
Human Forward: 5'-cca-ccg-gcg-gta-ctt-atg-aga-tcc-agt-cct-ggc-3' SEQ ID NO:1
Human Reverse: 5'-gct-agc-tca-gga-act-ttc-act-tcc-gtg-3'. SEQ ID NO:2.

The murine IL-21 primers used were as follows:
Murine Forward: 5'-cca-ccg-gcg-ggt-ggc-atg-gag-agg-acc-ctt-gtc-3' SEQ ID NO:3
Murine Reverse: 5'-gct-agc-cta-gga-gag-atg-ctg-atg-aat-3' SEQ ID NO:4

The PCR-amplified DNA fragments were cloned into TA Cloning vectors. One clone from human IL-21 and three clones from mouse IL-21 were obtained. These clones were verified by DNA sequencing. The one human IL-21 clone had two mutations in its sequence. Two of the murine clones had the correct sequence, and the third clone had 1 mutation in its sequence. The murine IL-21 cDNA fragments with the correct sequences were digested with *Sgr AI* and *Nhe I*, and cloned into the pORF5-mcs vector (InvivoGen; San Diego, CA). A large preparation of plasmid DNA was isolated using the Endofree™ Plasmid Mega purification kit by Qiagen, Inc. (Valencia, CA).

Example 2

This example demonstrates the cloning of murine IL-21.

Freshly isolated murine splenocytes from C57BL/6 mice were activated with 5 ng/ml PMA and 250 µg/ml ionomycin for 24 hr. Total RNA was extracted using TRIZOL (Invitrogen/Life Technologies). RT-PCR was performed to amplify the first strand of cDNA by random primers using ThermoScript RT-PCR System (Invitrogen/Life Technologies). The full-length mIL-21 cDNA fragment was PCR amplified using PCR SuperMix High Fidelity (Invitrogen/Life Technologies) and the primers of SEQ ID NOS: 3 and 4. The full-length murine IL-21 cDNA fragment was digested and cloned into the pORF-mcs vector under the control of an elongation factor-1 a/human T-cell leukemia virus (EF-lo/HTLV) hybrid promoter (InvivoGen, San Diego, CA), and was designated as pORF/mIL-21. The correct sequence of murine IL-21 was confirmed by sequence analysis. To exclude endotoxin contamination, a large preparation of pORF/mIL-21 and the control pORF plasmid DNA was purified using the EndoFree Plasmid Mega Kit (QIAGEN, Valencia, CA).

Example 3

This example demonstrates a method of administering to a mammal an IL-21 polynucleotide contained within an expression vector and an analysis of IL-21 expression in the mammal.

Injection of plasmid DNA encoding mIL-21 or control vector pORF-mcs was performed using the hydrodynamics-based gene delivery technique described by Liu et al., Gene Therapy 10,1735-1737 (1999), and Zhang et al., Human Gene Ther. 8, 71-74 (2000). Briefly, 8 to 10 week old mice were intravenously injected with 2 ml of saline containing varying amounts of plasmid DNA in 5 to 7 sec using a 25-gauge needle. The volume of solution injected was based on the age and weight of mice, and did not exceed 10% of body weight. Mice tolerated this treatment regimen well without obvious side effects observed after injection.

An ELISA system was used to detect mIL-21 expression in mouse serum. Briefly, monoclonal antibodies against murine IL-21 as a capture antibody were coated overnight onto a 96-well plate at 4 °C. Serial dilutions of serum samples were added to the coated plate the next day and incubated at 4 °C overnight. A biotin-labeled rat anti-mouse IL-21 polyclonal antibody was used as a detection antibody using standard methods (IL-21 antibodies were from R&D Systems).

This method allowed the prolonged production of large amounts of protein by hepatocytes following injection of plasmid DNA and was highly dependent on the volume and speed of injection. To determine the optimal promoter for our studies, we first compared the *in vivo* expression levels of a reporter gene, the chemokine GRO-α, in constructs with different promoters including LTR, CMV, and an EF-lcc/HTLV hybrid were compared. The EF-lcc/HTLV hybrid promoter generated the highest expression of the transgene *in vivo* after intravenous administration of plasmid DNA. This vector (pORF-mcs) was subsequently used for the mIL-21 *in vivo* antitumor studies.

A full-length murine IL-21 gene including a signal sequence was amplified by RT-PCR from activated murine splenocytes and subsequently ligated into pORF-mcs. The time course of mIL-21 expression in mouse serum following direct injection of pORF/mIL-21 plasmid DNA into the tail vein was then determined. As shown in Fig. 1, one day after a single dose of 20 µg of pORF/mIL-21 plasmid, a high level of mIL-21 was detected in mouse serum (6107 ± 2319 pg/mL) by a sandwich double antibody ELISA. Serum levels of murine IL-21 decreased over time, but were still as high as 278 ± 279 pg/mL on day 5, and returned to baseline on day 8. No detectable mIL-21 was seen in sera from naive mice or mice injected with the same amount of control plasmid DNA.

To determine the influence of IL-21 expression on immune cell populations *in vivo,* FACS analysis of mouse splenocytes was performed following plasmid administration. C57BL/6 mice were intravenously injected with 20 (ig of either pORF or mIL-21 DNA plasmid. Seven days later, mouse spleen cells were harvested and total cell numbers were counted. Cell suspensions were stained with fluorochromes conjugated specific antibodies and subjected to FACS analysis. As shown in Table 1, seven days after a single dose of 20 µg mIL-21 plasmid, the percentage of CD3⁺ and CD8⁺ T cells in the spleen was significantly increased with mIL-21-treated groups compared to the pORF control groups (51.3 ± 2.2% vs. 39.3 ± 5.3% and 26.8 ± 0.9% vs. 19.8 ± 4.1%, respectively; p = 0.0219 and 0.0418, respectively). Moreover, the percentage of cells in the myelomonocytic lineage as defined by CD lib and Gr-1 staining in the spleen was also significantly increased following mIL-21 administration (14.0 ± 0.7% vs. 31.4 ± 0.6%, and 11.4 ± 1.0% vs. 18.5 ± 1.6%, respectively; p < 0.0001 and p = 0.0027, respectively). However, the percentage of mouse NK cells, as defined by NK1.1+/CD3" or DX5+/CD3" subpopulations, from spleen was significantly decreased in the mIL-21-treated group compared to the pORF control group (3.0 ± 0.3% vs. 0.7 ± 0.1% and 3.6 ± 0.3% vs. 1.3 ± 0.2%, respectively; p = 0.0002 and 0.0001, respectively). Similar changes in the phenotype of immune cells comparable to those seen in splenocytes were observed in mouse peripheral blood. Since the spleen increased in size, weight, and total cell number following mIL-21 plasmid administration, the increase in the absolute number of T-cell and myelomonocytic cell subpopulations was even more profound in mIL-21-treated mice compared to control mice (Table 1). These observations suggested that the functional expression of mIL-21 *in vivo* after DNA injection had multiple biological effects on murine immune cells.

**Table 1: Effect of mIL-21 on immune cells in mouse spleens.**

| | % of positive cells | | Total no. of cells (x10⁶) | |
|---|---|---|---|---|
| | pORF | mIL-21 | pORF | mIL-21 |
| CD3 | 39.3 ±5.3 | 51.3 ±2.2* | 22.6 ±1.8 | 45.3 ±8.3* |
| CD4 | 26.0±1.5 | 28.1 ±2.4 | 15.0±1.2 | 24.9 ± 5.6* |
| CDS | 19.8 ±4.0 | 26.8 ±0.9* | 11.3±1.6 | 23.6 ±3. 8* |
| NK1.1⁺/CD3- | 3.0±0.3 | 0.7±0.1* | 1.7 ±0.4 | 0.7 ±0.2* |
| DX5⁺/CD3' | 3.6 ±0.3 | 1.3 ±0.2* | 2.1 ±0.5 | 1.1 ±0.3* |
| B220 | 63.2 ±5.2 | 53.2 ±2.7* | 36.8 ±7.7 | 46.6 ±6.4 |
| CD11b | 14.0±0.7 | 31.4±0.6* | 8.1 ±1.3 | 27.6±4.1* |
| CD11c | 7.8 ±0.4 | 9.6 ±1.2 | 4.5 ±0.8 | 8.4 ±0.8* |
| Gr-1 | 11.4±1.0 | 18.5 ±1.6* | 6.6 ±0.9 | 16.2 ±1.3* |

| | | | | |
|---|---|---|---|---|
| Three mice were in each group. Data represent 1 of 5 independent experiments with similar results. * indicates differences between pORF and mIL-21 groups were significant (p < 0.05). | | | | |

This example demonstrated that the administration of mIL-21 results in high levels of expression *in vivo* and that the expression alters splenocyte subpopulations.

Example 4

This example demonstrates a method of treating cancer in a mammal through the administration of an IL-21 polynucleotide.

On day 0, 8-10 week old C57BL/6 mice (NCI/Frederick, MD) were subcutaneously inoculated with 5 x 10⁵ B16 melanoma or MCA205 fibrosarcoma tumor cells. On day 5, tumor-bearing mice were intravenously injected with plasmid DNA dissolved in 2 mL of saline pre-warmed to room temperature. Seven days later, the DNA injection was repeated. Mice were ear tagged, and tumor growth rate was determined by blindly measuring tumors by perpendicular diameters 2 or 3 times per week using a digital caliper. The mouse survival rate was also recorded.

To study whether systemic expression of IL-21 can inhibit tumor growth in vivo, mIL-21 plasmid was injected 5 days after subcutaneous tumor implantation and repeated 7 days later, based on the mIL-21 expression time course. The response of a fibrosarcoma tumor line, MCA205, to increasing doses of mIL-21 was first determined. As shown in Fig. 2, all doses of plasmid DNA from 5 to 20 µg inhibited 5-day subcutaneous MCA205 tumor growth in a dose-dependent fashion with a maximum inhibition of 55% at a 20 µg dose level of mIL-21 plasmid (183 ± 25 vs. 410 ± 37 mm2, p = 0.0039) on day 31. Administration of the same amount of control pORF DNA did not have any effect on tumor growth. Importantly, no obvious toxic effects were observed in treatment mice exposed to this high level of mIL-21. In comparison, tumor-bearing mice that were injected with 1 µg of murine IL-2 DNA, which is the maximum tolerable IL-2 plasmid dose in mice, exhibited no antitumor effect (Fig. 2).

To determine whether the treatment effect of IL-21 was applicable to other types of tumors, B16 melanoma, a weakly immunogenic and more aggressive tumor was treated with mIL-21 in a 5-day subcutaneous model. As shown in Fig. 3, mIL-21 treatment significantly inhibited B16 melanoma growth *in vivo* (Fig. 3a, p < 0.0001). Of the 5 mice treated with mIL-21 in this experiment, 2 had a complete regression of tumor, and the other 3 had much smaller tumors compared to the control group in which all 5 mice had large tumors. The survival of mIL-21-treated B16-bearing mice was also significantly longer than that of control mice (Fig. 3b, p = 0.0031). On day 25 after tumor inoculation, all mice in the control group died, whereas 80% of mice in the treatment group were still alive. This experiment was repeated 3 times with similar results. Similar antitumor effects of IL-21 on another colon carcinoma tumor line, MC38, were also observed (data not shown).

This example demonstrated that IL-21 significantly inhibits tumor growth *in vivo* and prolongs survival.

Example 5

This example demonstrates that administration of IL-21 polynucleotide does not directly inhibit tumor growth *in vitro.*

The growth inhibition of tumor cells *in vitro* was determined by a 72-h MTS assay using the CellTiter 96Aqueous One Solution Assay kit according to manufacturer's instruction (Promega, Madison, WI). Briefly, 1x10⁵ murine tumor cells, including MCA205, B16, 24JK and MC38, were plated in 24 well plates in 1 mL of RPMI complete medium in combination with various amounts of recombinant mIL-21 protein. After 3 days, 100 µL of culture medium from each well were collected and incubated with 20 µL of MTS reagent at 37°C for 2 hr. Absorbance at 490 nm was then measured to determine the relative cell growth between groups.

IL-2 administration is known to upregulate multiple cytokines and hydrodynamics-based gene delivery can itself upregulate IL-12 and TNF-α (Hoffman et al., Gene Ther. 8, 71-74 (2000)). To determine whether recombinant IL-21 protein exhibits a direct inhibitory effect on the tumor cells used in the *in vivo* antitumor experiments, a 72-h MTS tumor growth inhibition assay was performed. As shown in Fig. 4, in the range of 20 to 100 ng/mL, recombinant mIL-21 did not exhibit any direct inhibitory effect on 4 tumor lines tested including MCA205 and B 16, none of which expresses IL-21R as determined by RT-PCR. In addition, FACS analysis of tumor cells for annexin V indicated no increase in tumor apoptosis following IL-21 treatment. These results suggest that IL-21 does not exhibit a direct inhibitory or cytotoxic effect on the tumor cells used in these studies, and other mechanisms, such as stimulation of immune cells, accounted for the observed *in vivo* antitumor activity.

This example demonstrated that mIL-21 does not directly inhibit tumor growth *in vitro.*

Comparative Example 6

This example demonstrates that IL-21 does not induce secretion of other cytokines.

C57BL/6 mice were injected intravenously with 20 µg of either pORF or pORF/mIL-21 plasmid DNA, or saline alone. Positive control mice were injected with 1 µg of mIL-2, mIL-4, mIL-10, and mIL-12 plasmid DNA, respectively. One, 4 and 8 days following injection, mice were sacrificed, and serum was collected and used to determine multiple cytokines (Pierce/Endogen, Woburn, MA).

To determine if IL-21 induced the secondary secretion of other cytokines that can have contributed to the antitumor response, serum samples were tested for a number of cytokines, including IL-1β, IL-2, IL-4, IL-5, IL-6, IL-10, IL-12, IFN-γ and TNF-α, using a multiple cytokine immunoassay. One, 4 and 8 days after a single injection of either of the mIL-21 or pORF plasmids (20 µg each) or saline, none of the cytokines tested, particularly IL-2, IL-12, IFN- y and TNF-α, which are known to have antitumor effects, was consistently elevated. Modest elevations in IL-6, IL-10 and IFN- y in mIL-21-treated mice were observed only on day 8, which were due to higher levels in only 1 of the 3 mice tested in that group, as seen by the high standard deviation for these values. Serum samples from mice that were injected with mIL-2, mIL-4, mIL-10, and mIL-12 plasmid DNA served as positive controls and all showed high levels of the corresponding cytokines (Table 2). These results indicate that the antitumor effects of IL-21 are not mediated by these cytokines.

**Table 2: Multiple cytokine secretion (pg/mL serum) in serum from mice injected with mIL-21.**

| Treatment | Day | IL-1b | IL-2 | IL-4 | IL-5 | IL-6 | IL-10 | IL-12 | IFN^y | TNFcc |
|---|---|---|---|---|---|---|---|---|---|---|
| Saline | 1 | 108 + 24 | 258+7 | 20+4 | 68+35 | 160+29 | 34+10 | 38+6 | **3**3+7 | 50+21 |
| pORF | 1 | 137+57 | 288+62 | 24+9 | 86+45 | 308 + 204 | 335 + 469 | 48+21 | 51 +24 | 75 + 37 |
| mIL-21 | 1 | 155+60 | 297 + 34 | 28+6 | 111+37 | 296+54 | 248+32 | 46+9 | 60+8 | 112+51 |
| None | 1 | 89+31 | 275+11 | 18+1 | 43 + 19 | 206 + 158 | 49+40 | 43+8 | 50+36 | 307 + 488 |
| Saline | 4 | 161 +83 | 393 + 102 | 39+9 | 151 +61 | 279 + 149 | 85+36 | 55+20 | 70 + 16 | 139+44 |
| pORF | 4 | 161 +36 | 292+73 | 25+7 | 66+17 | 124+27 | 47 + 18 | 53+8 | 49+8 | 78+45 |
| mIL-21 | 4 | 185+138 | 306 + 66 | 31+11 | 82+44 | 154 + 76 | 276+146 | 58+32 | 50+26 | 81 +31 |
| Saline | 8 | 232+281 | 205+170 | 22+15 | 95 + 47 | 146 + 86 | 44+24 | 36+25 | 40+30 | 83+36 |
| pORF | 8 | 137+20 | 276 + 27 | 28+4 | 90+19 | 130+15 | 50+9 | 44+24 | 48+11 | 78+22 |
| mIL-21 | 8 | 154+111 | 236 + 177 | 24+15 | 95+15 | 834 + 1269 | 413+489 | 52+37 | 253+375 | 98+30 |
| mIL-2 | 1 | 216 | 36935 | 50.8 | 532.8 | 1965 | 116.7 | 25 | 101.2 | 75 |
| mIL-4 | 1 | 69 | 61 | 1427 | 161 | 1820 | 550 | 23 | 71 | 142 |
| mIL- 10 | 1 | 49 | 176 | 40815 | 224 | 733.8 | 14935 | 27 | 270 | 681 |
| mIL-12 | 1 | 56 | 30 | 8.8 | 822 | 351 | 84 | 3990 | 222.1 | 67 |

| | | | | | | | | | | |
|---|---|---|---|---|---|---|---|---|---|---|
| Three mice were in each group at each point. Positive controls were from serum samples collected from mice injected with 1 µg of mIL-2, mIL-4, mIL-10, and mIL-12 plasmid DNA, respectively. Data represent 1 of 2 experiments with similar results. | | | | | | | | | | |

This example demonstrated that IL-21 does not induce secretion of other cytokines.

Example 7

This example demonstrates that NK cells are involved in the antitumor activity induced by mIL-21.

*In vivo* CD4 and CDS depletion was performed as described previously using anti-mouse CD4 (GK1.5) and CDS (2.43) antibodies (Wang et al., Nature Med. 4, 168-172 (1998)). Briefly, 2 and 4 days after tumor inoculation, tumor-bearing mice were intravenously injected with 200 µg/mouse of either anti-CD4 or CDS antibodies. The antibody injection was repeated intraperitoneally every 6 or 7 days thereafter during the experiment to maintain the depletion of CD4 and CDS cells. Murine IL-21 plasmid injection was performed on days 5 and 12. CD4 and CDS knockout mice (The Jackson Laboratory, Bar Harbor, ME) were also used for similar studies. Additional mice were included for each depletion study to verify the depletion of CD4 and CDS cells by FACS analysis. For *in vivo* NK cell depletion, anti-asialo GM1 antibody was used according to manufacturer's instruction (Wako Pure Chemical Industries). Briefly, anti-NK antibody was intravenously injected into tumor-bearing mice at 2 and 4 days after tumor inoculation, and repeated every 6 days intraperitoneally thereafter throughout the experiment to maintain the depletion. Tumor treatment was started on day 5 and repeated 7 days later.

Apoptosis was assessed by FACS staining of splenocytes using an Annexin V Apoptosis Detection kit from BD/PharMingen according to manufacturer's instructions.

The cytolytic activity of NK cells was determined by a standard ⁵¹Chromium-release assay. Briefly, the effector splenocytes isolated from mice injected with either mIL-21 or pORF plasmid DNA (4 days after injection) were incubated with ⁵¹Cr-labeled YAC-1 target cells at different E:T ratios at 37 °C for 4 hr.

Since the injection of mIL-21 resulted in an expansion of CD4⁺ and CD8⁺ lymphocytes in the spleen (Table 1) and peripheral blood, CD4⁺ or CD8⁺ T cells were depleted *in vivo* using specific monoclonal antibodies to determine if T cells are involved in mediating mIL-21-induced tumor regression. Murine IL-21-treated mice depleted of either CD4⁺ or CD8⁺ T cells still exhibited significant inhibition of MCA205 tumor growth, suggesting that T cells are not involved in IL-21 antitumor activity in this model (Fig. 5a, 5b and 5d). Tumor growth rate was noticeably higher in CDS-depleted mice compared to either CD4-depleted mice or control mice in the absence of mIL-21 (Fig. 5a, 5b and 5d), indicating that endogenous CD8⁺ T cells can have some inhibitory effects on the baseline tumorogenicity of MCA205, a weakly immunogenic tumor line. Nevertheless, with the addition of mIL-21 plasmid, MCA205 tumor growth was significantly inhibited, indicating that IL-21 could work through a CDS-independent mechanism. Indeed, as shown in Fig. 5c, the antitumor activity of mIL-21 was completely abolished after *in vivo* depletion ofNK cells. These experiments demonstrate that the inhibitory effect of mIL-21 on MCA205 tumor is mainly mediated through NK cells. This was further supported by a similar experiment using either CD4 or CDS knockout mice, which showed that mIL-21 could induce tumor regression in the absence of CD4⁺ or CD8⁺ T cells. However, there is still a possibility that CD8⁺ T cells can play a partial role in this antitumor effect, since tumor growth rate in mIL-21-treated mice is greater in CDS-depleted mice compared to control mice (Fig. 5b and 5d).

Interestingly, as shown in Table 1, the percentage and total number of NK1.1+/CD3" or DXS+/CD3" splenic NK cells actually decreased after mIL-21 injection compared to mice injected with pORF control vector. To further investigate the mechanism of this decrease, NK cell apoptosis was assessed following *in vivo* mIL-21 plasmid injection. As shown in Fig. 6a, annexin V staining of NK1.1+/CD3" splenic NK cells increased from 16.9% to 41.6% 4 days after mIL-21 plasmid injection, indicating that mIL-21 had an apoptotic effect on NK cells *in vivo.* To evaluate NK cell activity following IL-21, ⁵¹Chromium-release assays were performed against the NK target YAC-1 using splenic NK cells. Four days after *in vivo* mIL-21 plasmid injection, splenic NK cell lysis against YAC-1 was significantly increased (Fig. 6b). Similar observations were also seen in IL-21 transgenic mice (data not shown). These results demonstrate that IL-21 *in vivo* can induce NK cell apoptosis, while enhancing activation and lytic ability, which can explain the observed NK-dependent antitumor activity in mIL-21-treated mice, despite a reduction in the number of splenic NK cells.

This example demonstrated that NK cells are involved in the antitumor activity induced by mIL-21.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising, " "having, " "including, " and "containing" are to be construed as open-ended terms (i.e., meaning "including, but not limited to, ") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments can become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. Use of an IL-21 polypeptide as shown in SEQ ID NO: 5 or 7 or a fragment thereof, wherein the fragment has the biological activity of IL-21, in the preparation of a medicament for treating or preventing cancer in a mammal.

2. Use of an IL-21 polynucleotide as shown in SEQ ID NO: 6 or 8 or a fragment thereof having the biological activity of IL-21 in the preparation of a medicament for treating or preventing cancer in mammal.

3. Use of an expression vector containing an IL-21 polynucleotide as shown in SEQ ID NO: 6 or 8 or a fragment thereof having the biological activity of IL-21 in the preparation of a medicament for treating or preventing cancer in a mammal.

4. The use according to claim 3, wherein the expression vector is pORF.

5. The use according to any of claims 1-4, wherein the cancer is a melanoma, a sarcoma, or a colon cancer.

6. The use according to claim 1, wherein the IL-21 polypeptideor fragment thereof is used together with a vaccine, an antigen-specific T lymphocyte, a cytokine, or a combination thereof in the preparation of the medicament.

7. The use according to claim 2, wherein the IL-21 polynucleotide or fragment thereof is used together with a vaccine, an antigen-specific T lymphocyte, a cytokine, or a combination thereof in the preparation of the medicament.

8. The use according to claim 3, wherein the expression vector is used together with a vaccine, an antigen-specific T lymphocyte, a cytokine, or a combination thereof in the preparation of the medicament.

9. The use according to any of claims 6-8, wherein the vaccine is a recombinant viral vaccine or a peptide vaccine.

10. The use according to any of claims 6-8, wherein the cytokine is IL-2, IL-7, or IL-15.

11. The use according to any of claims 6-8, wherein the antigen-specific T lymphocyte is a tumor specific T lymphocyte.

## Patentansprüche

1. Verwendung eines IL-21- Polypeptids, wie in SEQ ID NR: 5 oder 7 gezeigt, oder ein Fragment davon, wobei das Fragment die biologische Aktivität des IL-21 aufweist, zur Herstellung eines Medikaments zur Behandlung oder Vorbeugung von Krebs in einem Säuger.

2. Verwendung eines IL-21- Polynukleotids wie in SEQ ID NR: 6 oder 8 gezeigt, oder ein Fragment davon, das die biologische Aktivität des IL-21 aufweist, zur Herstellung eines Medikaments zur Behandlung oder Vorbeugung von Krebs in einem Säuger.

3. Verwendung eines Expressionsvektors, der ein IL-21- Polynukleotid enthält wie in SEQ ID NR: 6 oder 8 gezeigt, oder ein Fragment davon, das die biologische Aktivität des IL-21 aufweist, zur Herstellung eines Medikaments zur Behandlung oder Vorbeugung von Krebs in einem Säuger.

4. Verwendung nach Anspruch 3, wobei der Expressionsvektor pORF ist.

5. Verwendung nach einem der Ansprüche 1-4, wobei der Krebs ein Melanom, ein Sarkom oder ein Darmkrebs ist.

6. Verwendung nach Anspruch 1, wobei das IL-21- Polypeptid oder Fragment davon zusammen mit einem Vakzin, einem Antigen-spezifischen T-Lymphozyten, einem Zytokin oder einer Kombination davon bei der Herstellung eines Medikaments verwendet wird.

7. Verwendung nach Anspruch 2, wobei das IL-21- Polynukleotid oder Fragment davon zusammen mit einem Vakzin, einem Antigen-spezifischen T-Lymphozyten, einem Zytokin oder einer Kombination davon bei der Herstellung eines Medikaments verwendet wird.

8. Verwendung nach Anspruch 3, wobei der Expressionsvektor zusammen mit einem Vakzin, einem Antigen-spezifischen T-Lymphozyten, einem Zytokin oder einer Kombination davon bei der Herstellung eines Medikaments verwendet wird.

9. Verwendung nach einem der Ansprüche 6-8, wobei das Vakzin ein rekombinantes virales Vakzin oder ein Peptid-Vakzin ist.

10. Verwendung nach einem der Ansprüche 6-8, wobei das Zytokin IL-2, IL-7 oder IL-15 ist.

11. Verwendung nach einem der Ansprüche 6-8, wobei der Antigen-spezifische T-Lymphozyt ein Tumor-spezifischer T-Lymphozyt ist.

## Revendications

1. Utilisation d'un polypeptide IL-21 tel que présenté dans SEQ ID N° : 5 ou 7 ou d'un fragment de celui-ci, le fragment ayant l'activité biologique de IL-21, dans la préparation d'un médicament pour traiter ou prévenir le cancer chez un mammifère.

2. Utilisation d'un polynucléotide IL-21 tel que présenté dans SEQ ID N° : 6 ou 8 ou d'un fragment de celui-ci ayant l'activité biologique de IL-21, dans la préparation d'un médicament pour traiter ou prévenir le cancer chez un mammifère.

3. Utilisation d'un vecteur d'expression contenant un polynucléotide IL-21 tel que présenté dans SEQ ID N° : 6 ou 8 ou d'un fragment de celui-ci ayant l'activité biologique de IL-21, dans la préparation d'un médicament pour traiter ou prévenir le cancer chez un mammifère.

4. Utilisation selon la revendication 3, dans laquelle le vecteur d'expression est pORF.

5. Utilisation selon l'une quelconque des revendications 1 à 4, dans laquelle le cancer est un mélanome, un sarcome ou un cancer du côlon.

6. Utilisation selon la revendication 1, dans laquelle le polypeptide IL-21 ou le fragment de celui-ci est utilisé conjointement avec un vaccin, un lymphocyte T spécifique d'un antigène, une cytokine ou une combinaison de ceux-ci dans la préparation du médicament.

7. Utilisation selon la revendication 2, dans laquelle le polynucléotide IL-21 ou le fragment de celui-ci est utilisé conjointement avec un vaccin, un lymphocyte T spécifique d'un antigène, une cytokine ou une combinaison de ceux-ci dans la préparation du médicament.

8. Utilisation selon la revendication 3, dans laquelle le vecteur d'expression est utilisé conjointement avec un vaccin, un lymphocyte T spécifique d'un antigène, une cytokine ou une combinaison de ceux-ci dans la préparation du médicament.

9. Utilisation selon l'une quelconque des revendications 6 à 8, dans laquelle le vaccin est un vaccin viral recombinant ou un vaccin peptidique.

10. Utilisation selon l'une quelconque des revendications 6 à 8, dans laquelle la cytokine est IL-2, IL-7 ou IL-15.

11. Utilisation selon l'une quelconque des revendications 6 à 8, dans laquelle le lymphocyte T spécifique d'un antigène est un lymphocyte T spécifique d'une tumeur.
